# EUROPEAN PATENT APPLICATION

(11) **EP 4 321 630 A1**
(43) Date of publication of application: **14.02.2024**
(21) Application number: 22189451.2
(22) Date of filing: 09.08.2022
(51) Int. Cl.: C12Q 1/6855, G16B 30/20

(54) **METHOD OF PARALLEL, RAPID AND SENSITIVE DETECTION OF DNA DOUBLE STRAND BREAKS**

(71) Applicant: Institute of Molecular Biology gGmbH (IMB), 55128 Mainz (DE)
(72) Inventor: Roukos, Vassilis, 55122 Mainz (DE); Mello da Cunha Longo, Gabriel, 55122 Mainz (DE); Sayols Puig, Sergi, 17844 Cornella del Terri (ES)
(74) Representative: Moré, Solveig Helga

(57) **Abstract**

The present invention relates to the field of gene editing and methods for its optimization and control. In particular, the present invention provides a method for detecting one or more double-strand breaks (DSBs) in DNA. The method of the invention is designated BreakTag and relies on directly labelling DSB ends. It is preferably used for discovering on- and/or off-targets of genome-editing nucleases, in particular CRISPR nucleases such as Cas9, Cas12 and variants thereof *in vitro,* in cells or in living organisms. In a core step, it uses a PCR suppression step to enrich for DSBs via tagmentation. The invention further provides a method for determining whether a CRISPR nuclease coupled with a specific gRNA generates a DNA DSB having blunted or a staggered ends. Finally, the invention provides kits and computer program products suitable for performing said methods.

## Description

The present invention relates to the field of gene editing and methods for its optimization and control. In particular, the present invention provides a method for detecting one or more double-strand breaks (DSBs) in DNA. The method of the invention is designated BreakTag and relies on directly labelling DSB ends. It is preferably used for discovering on- and/or off-targets of genome-editing nucleases, in particular CRISPR nucleases such as Cas9, Cas12 and variants thereof *in vitro,* in cells or in living organisms. In a core step, it uses a PCR suppression step to enrich for DSBs via tagmentation. The invention further provides a method for determining whether a CRISPR nuclease coupled with a specific gRNA generates a DNA DSB having blunted or a staggered ends. Finally, the invention provides kits and computer program products suitable for performing said methods.

Genome engineering using the CRIPSR/Cas system holds immense promise to treat or even cure genetic disorders and shows great potential in diagnostics and biotechnology of agriculture. For example, the method has been used to generate crops resistant to cold, heat and herbicides. Moreover, CRISPR has been successfully employed to reverse genetic diseases in pre-clinical and Phase I trials by modifying defective genes in patients, and it is expected to be used in the treatment of several diseases in the next few years. In spite of its promising role in the era of precision medicine, many studies have reported that CRISPR nucleases can cleave numerous off-target sites across the genome depending on the target sequence, raising concern with the translation of CRISPR into the clinic (Sharma et al., 2021). In the case of some CRISPR nucleases, these off-target cleavage events result in the formation of DNA double-strand breaks (DSBs), which, dependent on their location, cause severe genomic instability in the form of mutations and rearrangements, and may lead to serious unwanted effects including miss-regulation of cancer-associated genes.

Given the central role that CRISPR will have in personalized medicine in the next years, there is a demand for tools that can map off-target effects. Different methods were generated over the past five years that allow for the interrogation of CRISPR off-targets *in vitro,* in cells or *in vivo,* but a unifying tool for the gRNA safety at different levels without changing the workflow is lacking (Atkins et al., 2021).

Previously developed tools can be divided into two major groups: Cell-free DNA *(in vitro)* methods and cell-based methods, each with their own set of advantages and disadvantages. Cell-free methods rely on the *in vitro* digestion of genomic DNA (gDNA) with RNPs (ribonucleoprotein, Cas9+sgRNA), where cleavage events are enriched, sequenced and off-targets are called based on short read sequencing signal pileup. Common enrichment strategies are labelling free DSB ends with biotin linkers followed by a streptavidin pull-down (SITE-seq) (Cameron et al., 2017), or circularization the entire gDNA and selective linearization of circles that contain cleaved sequences, making them amenable to sequencing (CIRCLE-seq) (Tsai et al., 2017). However, these methods are labor-intensive and cannot be performed in a high-throughput manner. CHANGE-seq improves on CIRCLE-seq, and allows high-throughput nomination of CRISPR off-targets by applying tagmentation using Tn5, reducing the sample input and hands-on time, but it cannot detect cleavage events by Cas12 and other nucleases that will likely have an important role in the near future (Lazzarotto et al., 2020). Nonetheless, circularization methods cannot capture DSBs generated in living cells. In general, *in vitro* methods will yield a larger list of off-targets, as the chromatin context will not be taken into account, and not every region available for cleavage *in vitro* is accessible to CRISPR when the chromatin context is in place.

Methods that rely on the integration of donor sequences at DSB sites, such as GUIDE-seq (Tsai et al., 2015) and TTISS-seq (Schmid-Burgk et al., 2020) are the most sensitive group of tools in off-target discovery in living cells. One of the drawbacks of such tools is that integration of donor sequences takes place in sites repaired by end-joining mechanisms, and can presumably miss sites of DSBs repaired by other pathways (Atkins et al, 2021). It is worth mentioning that hard-to-transfect cells are prohibited to such tools, and electroporation of the donor sequence was shown to be toxic to iPS cells (Wienert et al., 2020). Moreover, integration-based tools cannot be used for off-target discovery in living organisms.

Another study published in Science in 2020 reported DISCOVER-seq, a method that uses Chromatin immunoprecipitation followed by sequencing (ChIP-seq) for the repair factor MRE11 as a surrogate for CRISPR off-targets (Wienert et al., 2020). DISCOVER-seq mapped off-targets in cells extracted from tissues of edited mice, showing its applicability in mapping unintended Cas9 and Cas12 breaks in living organisms. The major drawback of this method is the relatively high amount of starting material required, the lack of scalability and the laborious protocol. Previous methods such as End-seq (Canela et al., 2016) BLISS (Yan et al., 2017) and BLESS (Crosetto et al., 2013) have been validated for the detection of CRISPR off-targets, but their costly and labor-intensive workflow that requires days/weeks for completion prohibits up-scaling. In the following, Table 1 summarizes the above-mentioned methods known in the state of the art and provides an overview on their most important advantages and disadvantages.

**Table 1: Summary of known methods used for the interrogation of CRISPR off-targets**

| **Method** | **Enrichment method** | **Drawback** | **Strength** | **References** |
|---|---|---|---|---|
| **DIGENOME-seq** | - | Low sensitivity, lacks enrichment of cleavage events, low throughput and cost-inefficient | - | Kim et al., 2017 |
| **SITE-seq** | Biotin-streptavidin pull-down | High material input, protocol is labour-intensive, | High sensitivity | Cameron et al., 2017 |
| **CIRCLE-seq** | Circularization of genome, cleavage, and sequencing of linearized circles | High material input, protocol is labour-intensive and time-inefficient. Does not discriminate Cas9 DSB end structure | High sensitivity with minimal background | Lazzarotto et al., 2018 |
| **CHANGE-seq** | Circularization of genome, cleavage, and sequencing of linearized circles | Can be used only for *in vitro* nomination of Cas9 off-targets. Does not discriminate Cas9 DSB end structure | High-throughput, low sample input, time-efficient (when used together with a liquid handling platform) and low background | Lazzarotto et al., 2020 |
| **BLISS** | Labelling of DSB ends + T7 *in vitro* transcription | Protocol is expensive, labour-intensive and time consuming. | Low sample input | Yan et al., 2017; Gothe et al., 2019; Bouwman et al, 2020 |
| **GUIDE-seq** | Nested PCR for enrichment of integration events | Misses off-targets repaired by end-joining independent mechanisms, cannot be adapted for base editor off-target discovery, and cannot be used in animal or iPSC models. Does not discriminate Cas9 DSB end structure | Low sample input, excellent signal/noise ratio | Tsai et al., 2015 |
| **TTISS-seq** | Nested PCR for enrichment of integration events | Misses off-targets repaired by end-joining independent mechanisms, cannot be adapted for base editor off-target discovery and cannot be used in animal or iPSC models. Does not discriminate Cas9 DSB end structure | Low sample input, excellent signal/noise ratio and high-throughput | Schmid-Burgk et al., 2020 |
| **DISCOVER-seq** | Immunoprecipitation of MRE11, a repair factor, as a surrogate for CRISPR off-targets. | Indirect assessment of CRISPR off-targets, time-inefficient, labour intensive and can only be used in the nomination step | Can be used in animal models and stem-cells | Wienert et al., 2020 |

Collectively, a method that can capture the full range of CRISPR-nucleases off-targets *in vitro* and *in vivo* without changing the workflow in a high-throughput manner is desirable.

In light of the state of the art, the present inventors thus addressed the problem of providing a novel and highly sensitive high-throughput method for directly detecting DNA DSBs, overcoming many of the limitations of presently used methods, and which, advantageously, has the potential to profile unintended off-target sites of different CRISPR nucleases on a genome-wide level.

The problem is solved by the present invention, in particular by the subject matter of the claims. The method of the invention is designated BreakTag and relies on directly labelling DSB ends to discover CRISPR off-targets *in vitro,* in cells or in living organisms. This direct approach bypasses the need of integration events, immunoprecipitation and other indirect methods of CRISPR-breaks detection. The method according to the invention relies only on few enzymatic reactions, is "multiplex-friendly" and requires merely minimal hands-on time, which makes it easy to perform, easily scalable and inexpensive.

In particular, the present invention provides a method for detecting one or more double-strand breaks (DSBs) in DNA comprising steps of
a) providing DNA comprising one or more DSBs,
b) ligating a first linker to the DSB ends to obtain a first linker-DNA conjugate, wherein the first linker comprises a UMI ;
c) preferably, removing non-ligated first linkers;
d) enzymatically fragmenting the first linker-DNA conjugates and tagging the obtained DNA fragments with a second linker;
e) PCR-amplifying the tagged DNA fragments to fill in gaps in the DNA sequence of these DNA fragments to obtain a first heterotagged population of DNA fragments consisting of DNA fragments flanked by the first linker and the second linker and a second homotagged population of DNA fragments consisting of DNA fragments flanked by two second linkers and not comprising the first linker;
f) enriching the first heterotagged population of DNA fragments by PCR-amplifying and, optionally, size selecting the DNA fragments, wherein, during PCR amplification, a first and a second full-length sequencing adapter is attached to the first and second linker, respectively, at the ends of each DNA fragment of the first heterotagged population of DNA fragments to obtain a sequencing library;
g) sequencing the sequencing library comprising the first heterotagged population of DNA fragments; and
h) bioinformatically analyzing the sequencing results to detect the one or more DNA DSBs.

DNA (deoxyribonucleic acid) is a biopolymer formed of monomeric building blocks called nucleotides. Each nucleotide consists of the 5-carbon sugar deoxyribose, a phosphate group and one of the four nitrogenous bases adenine (A), guanine (G), cytosine (C) and thymine (T). DNA usually exists in a double-stranded state due to the hybridization of two single-stranded DNA molecules via complementary base pairing, i.e. the nitrogenous base adenine present in one DNA strand pairs and forms hydrogen bonds with thymine in the opposing strand, whereas cytosine pairs with guanine. As a result, DNA takes a characteristic double-helical shape in cells. Less than 5% of the total DNA in a cell codes for polypeptides, whereas the rest is believed to perform mainly regulatory functions during protein biosynthesis or no function at all. Within the nucleus of a eukaryotic cell, DNA is wrapped around complexes formed of histone proteins to create a structure known as chromatin that is further sub-divided into separate units known as chromosomes.

DNA double-strand breaks (DSBs) are often considered to represent the most severe type of DNA damage as they result in chromosome breakages that, if left unrepaired, may cause the death of a cell. On the other hand, misrepairing of DSBs can result in chromosomal rearrangements, such as deletions, inversions and translocations that could promote the development of various types of cancer. The two major pathways specialized on DSB repair represent non-homologous end joining (NHEJ) and homologous recombination-directed repair (HRR). While NHEJ is the main DSB repair pathway, it is considered to be a rather error-prone repair mechanism, as it simply re-ligates the two breakage ends, thereby often generating small deletions or insertions into the repaired DNA. By comparison, the error-free, yet more time-consuming HRR DSB repair relies on a homologous repair template to resolve DSBs. It therefore is restricted to particular phases of the cell cycle when a homologous DNA repair template is present, i.e., S and G2. The repair template may be a sister chromatid or a homologous chromosome.

In the context of the invention, "detecting" a DSB is understood to mean that the method of the invention enables the skilled person to understand whether a given DNA molecule comprises one or more DSBs or not. Detecting may also encompass quantifying DSBs, i.e., finding how many DSBs are present in the DNA that is to be analyzed. It may be further used for the comparison of DSB quantities, i.e., testing whether a DNA obtained from a given sample contains more or less DSBs than a DNA obtained from a different sample. In a preferred embodiment, the term "detecting" is further understood to mean that the precise location of a DSB within a given DNA is to be determined. The identification of the location of a DSB within a region or stretch of a DNA is essential in case the cleavage specificity of genome-editing CRISPR nucleases is to be determined, as will be described in more detail below.

The one or more DSBs that are to be detected with the method according to the invention may have originated in different ways. For instance, the one or more DSBs in the DNA may have been induced by a genome-editing nuclease, restriction enzymes, a chemical agent or radiation.

The term "genome-editing nucleases" refers to enzymes that are capable of cleaving the phosphodiester bonds between nucleotides of a nucleic acid in a programmable and therefore targetable fashion and can thus be used to introduce highly specific modifications into the sequence of a given nucleic acid. Genome-editing nucleases have emerged in recent years as an extremely popular tool for biomedical and biotechnical research. There are different types of genome-editing nucleases which are capable of inducing both single-strand breaks (SSBs), base conversions or DSBs. Preferably, the method of the invention is able to detect DSBs that have been directly induced by a genome-editing nuclease. However, under certain circumstances and experimental treatments, the DSBs that are detected by the present method may arise from SSB induced by particular types of genome-editing nucleases and/or conversions of certain bases induced by base editors. Accordingly, the method of the invention is also able to detect such kind of indirectly induced DSBs.

The genome-editing nuclease used to introduce one or more DSBs may be selected from the group comprising a CRISPR nuclease, a Meganuclease, a Zinc Finger Nuclease (ZNF), or a transcription activator-like effector nuclease (TALEN).

In a preferred embodiment, the one or more DSBs in the DNA have been induced by a CRISPR nuclease. In the context of the invention, "CRISPR nuclease" refers to a family of endonucleases also known as "CRISPR associated proteins" or simply "Cas" proteins. Accordingly the terms "CRISPR nuclease", "Cas protein", "Cas nuclease" or "CRISPR/Cas nuclease" are used interchangeably herein. CRISPR nucleases are RNA-guided enzymes that cleave DNA in a sequence-specific manner, acting like molecular scissors. In brief, CRISPR nucleases can be loaded with a so-called guide RNA sequence (gRNA). In the context of the invention, the gRNA typically is a single-guide RNA or sgRNA, i.e., a short synthetic RNA sequence that has been originally developed by artificially joining a CRISPR RNA (crRNA) and a trans-activating crRNA (tracrRNA), which naturally occur in bacteria as part of the antiviral adaptive immune system CRISPR (short for Clustered Regularly Interspaced Short Palindromic Repeats). The term "gRNA" may however also encompass embodiments in which the crRNA and tracrRNA are present as separate molecules. The gRNA comprises a specifically designed 20 nt "spacer" region at its 5' end that is capable of binding to a complementary target sequence of interest in a targeted DNA. In the context of the invention, the target sequence of a CRISPR nuclease is also referred to as "protospacer", although, strictly speaking, this term was originally used to merely refer to a stretch of DNA in a virus that is targeted by the endogenous bacterial CRISPR system. By binding to the target sequence or "protospacer", the gRNA directs the associated CRISPR nuclease to the specific location of the DNA that is to be cleaved. Cleavage of the DNA by the CRISPR nuclease further requires the target sequence to precede a so-called protospacer adjacent motif (PAM). The PAM is a short DNA sequence of about 2 to 6 base pairs that is located about 3 to 4 nucleotides downstream from the cut site.

Different RNA-guided CRISPR nucleases are used in gene editing approaches, with Cas9 being the most common one. Cas9 was first discovered in the bacteria *Staphylococcus pyogenes,* and once loaded with a gRNA sequence, will scan a DNA until it finds a PAM with the canonical sequence NGG. Cas9 then unwinds the DNA and if base complementarity between the gRNA and target DNA exists, a conformational change activates its nuclease domains, generating DSBs (Jiang and Doudna, 2017). Although Cas9 is the most commonly used nuclease in gene-editing experiments, numerous CRISPR nucleases have also been shown to work as molecular scissors. Cas12 (formerly, Cpf1) is another promising nuclease first discovered in bacteria of the *Acidaminococcus* genus. Cas12 can also generate DSBs with the advantage of generating staggered DSBs that can be harnessed to homology-directed repair (Doudna, 2020). Compared to Cas9, Cas12 has a smaller size and thus can be delivered to cells more easily.

Accordingly, the one or more DSBs in the DNA have been preferably induced by Cas9 or Cas12.

In the past, several variants of Cas9 and Cas12 have been developed and described in a quest to obtain a Cas protein with improved target specificity. Accordingly, the one or more DSBs that are to be detected by the method of the invention may also be induced by any of these variants of Cas9 or Cas12. The one or more DSBs may also be the indirect result of any CRISPR nuclease that is not Cas9 or Cas12 and normally does not directly induce DNA DSBs.

Before the discovery and advancement of the CRISPR/Cas system, other nucleases were already used for genome-editing experiments. For instance, meganucleases are endonucleases targeting double-stranded DNA that were originally discovered in yeast but were also identified in many other microbial genomes. They are characterized by a comparably large recognition site of 12 to 40 base pairs (bp), which means that the recognition site of any given meganuclease usually occurs not more than once in a genome. However, as of today, hundreds of naturally occurring meganucleases have been identified, which considerably expanded the choice of targetable DNA loci. Moreover, site-directed genome modification in mammalian cells has been achieved by using meganucleases with genetically altered DNA recognition sites (Tröder and Zevnik, 2022).

Zinc Finger (ZNF) nucleases represent another class of programmable nucleases capable of introducing DNA DSBs. Zinc fingers are protein motifs that are stabilized by zinc ions and are able to bind to DNA in a sequence-specific manner. The first ZNF nuclease was produced in 1996 by fusing an array consisting of several modules of such zinc fingers to the DNA cleavage domain of the restriction enzyme Fokl. Each of these zinc finger modules contained a DNA binding motif specific to three consecutive base pairs of the target sequence. Accordingly, this modular design permits relatively flexible modification of the DNA recognition site of ZNF nucleases (Tröder and Zevnik, 2022).

The principle of these ZNF nucleases was further advanced with the introduction of transcription activator-like effector nucleases (TALENs). In TALENs, the nuclease domain is not fused to a Zinc finger array but instead to a TAL targeting domain. The latter is derived from Transcription Activator-Like Effector (TALE) proteins discovered in plant pathogenic bacteria of the genus *Xanthomonas.* The TAL targeting domain consists of 33-35 amino acids repeats. Each of these repeats recognizes only one of the four nucleotides. Accordingly, the DNA binding modules of TALENS allow for easier modification and more flexible DNA recognition than those of ZNF nucleases (Tröder and Zevnik, 2022).

In an alternative embodiment, the one or more DNA DSBs are induced by a restriction enzyme. Restriction enzymes are endonucleases capable of cleaving double-stranded DNA into fragments within or at close proximity to a specific recognition sequence known as restriction site. The restriction site of a given restriction enzyme usually has a length of about 4 to 8 base pairs. Restriction enzymes are among the most important tools in molecular biology. Currently, more than 800 different restriction enzymes are commercially available. (https://en.wikipedia.org/wiki/Restriction_enzyme). Different restriction enzymes may induce either blunt DSBs or sticky-ended (i.e., staggered) DSBs comprising 5' or 3' overhangs.

DNA DSBs may alternatively be induced by chemical means. For instance, alkylating agents such as methyl methanesulfonate (MMS) cause DNA modification that may lead to replication fork collapse and, subsequently, to the occurrence of DNA DSBs. DNA DSBs may also arise indirectly upon exposure to DNA crosslinking agents such as mitomycin C, and Cisplatin or the chemotherapeutic Etoposide. Etoposide forms a complex with DNA and the enzyme topoisomerase II, thereby preventing the re-ligation of DNA DSBs that are naturally induced by the topoisomerase in order to remove DNA supercoils.

Finally, the DSBs that are to be detected by the method of the invention may be induced by irradiation, in particular ionizing radiation such as X-rays or γ-rays. However, exposure to ultraviolet (UV-) light may also indirectly induce DSBs in DNA, as it triggers the formation of helix-distorting adducts such as cyclobutane pyrimidine dimers (CPDs) and pyrimidine (6-4) pyrimidine photoproducts (6-4PPs), which can cause the stalling and collapse of replication forks.

The DNA that is to be analyzed by the method according to the invention may be any type of double-stranded DNA. In a preferred embodiment, the DNA is genomic DNA, i.e. DNA that is organized in the form of chromosomes and comprises an organism's hereditary information. In eukaryotes, the genomic DNA is stored in the cell nucleus. The DNA may however also be an extrachromosomal DNA, such as a mitochondrial DNA, a chloroplast DNA, a eukaryotic extrachromosomal circular DNA, a viral DNA or a plasmid. Accordingly, the DNA may be a eukaryotic DNA, a prokaryotic DNA or a viral DNA. In yet another embodiment, the DNA may be synthetically generated DNA. It may, e.g., be a complementary DNA (cDNA) produced by reverse transcribing an RNA or a DNA that has been generated by artificial gene synthesis.

The DNA comprising one or more DSBs in step a) may be obtained from an organism, a tissue or a cell. For instance, the DNA may be isolated from yeast, a plant or an animal, a plant tissue or animal tissue or a plant cell or animal cell. The yeast may be, e.g., from the genus *Saccharomyces* such as, e.g., *S*. *cerevisiae, S. carlsbergensis or S. pastorianus,* from the genus *Schizosaccharomyces* such as, e.g., *S*. *pombe,* or from the genus *Candida* such as, e.g., *C. albicans.* The plant may be, e.g., *A. Thaliana, N. tabacum,* a food or energy crop such as maize, wheat, rye, oats, barley, rice, sorghum, potato, sweet potato, manioc, sugar cane or soy.

The DNA may also be isolated from an animal, animal tissue or animal cell. The animal may be, e.g., a human, a primate, a rat, a mouse, a fly, a nematode or a fish.

The organism, tissue or cell may have been, e.g., exposed to DSB-inducing radiation or have been treated with a genome-editing nuclease such as a CRISPR nuclease. Likewise, the DNA may be isolated from a cell or a tissue in culture, which has been treated with, e.g., a genome-editing nuclease such as a CRISPR nuclease.

The DSBs may also have been induced *in vitro,* by any of the means described herein. In such an embodiment, the DNA that is provided in step a) of the method of the invention would be an isolated DNA that has been brought into contact with, e.g., the genome-editing nuclease, preferably the CRISPR nuclease *in vitro,* i.e., in a suitable reaction vessel such as a test tube.

If the DNA that is to be analyzed for the presence of DSBs using the method of the invention is obtained from an organism, a tissue or a cell treated with any of the DSB-inducing means described herein, it may, in some embodiments, be advantageous to impair or even completely disrupt one or more DSB repair pathways in said organism, tissue or cell. In doing so, repair of the DSBs can be delayed or even prevented, thus increasing the number of DSBs that are detectable by the present method. For example, the method may be performed on DNA that has been obtained from a knock-out mutant of a gene required for functional non-homologous end joining (NHEJ), such as, e.g., a Ku70 knock-out mutant, a Ku80 knock-out mutant or a Ligase IV knockout mutant. Alternatively or in addition, the DNA can be obtained from a knock-out mutant of a gene required for functional homologous recombination-directed repair (HRR), such as, e.g., a BRCA1 mutant or a BRCA2 mutant. In yet another embodiment, repair of DSBs may be impaired or disrupted in a transient manner, e.g., by using RNA interference against one or more genes required for DSB repair. The DNA may also be isolated from a transgenic cell or animal, in which the expression of one or more DNA DSB repair factors can be switched on or off, e.g., by means of an inducible promoter.

The method according to the invention can be performed with 1500 ng DNA or less, e.g., with 1400, 1300, 1200, 1100, 1000, 900, 800, 700, 600, 500, 400, 300, 200, or 100 ng DNA. Preferably, the method according to the invention is performed with 200 to 700 ng DNA, most preferably with 500 ng DNA.

In step b) of the method according to the invention, the ends of the one or more DSBs are ligated to a first linker. Before this step, it may however be necessary to process the DSB ends first. Indeed, most DNA double-strand breaks (DSBs) formed in a natural environment exhibit chemical modifications at or near the ends that interfere with the ligation of linkers. Moreover, the majority of DSB breakage ends, including those induced by many genome-editing nucleases, are staggered, i.e., possess single-stranded DNA overhangs. Similarly, homologous recombination repair (HRR) of DNA DSB involves an initial 5' end resection step of the DSB ends which results in short 3' overhangs. These single-stranded overhangs need to be trimmed or filled up to obtain blunt ends prior to the addition of the first linkers. Accordingly, in most embodiments, the method involves, after step a) and before step b), the enzymatic blunting of DSB ends to remove single-stranded DNA overhangs at the breakage sites. The blunting of DSB overhangs is preferably achieved using the 68 kDa E. *coli* DNA Polymerase I Large (Klenow) Fragment. This Klenow Fragment lacks the 5' → 3' exonuclease activity of intact DNA polymerase I, but retains its 5' → 3' polymerase and 3' → 5' exonuclease as well as its strand displacement activities. The 3' → 5' exonuclease activity is able to generate blunt ends by cutting back a 3'-overhang. Alternatively, in case a DSB end exhibits a 5' overhang, the Klenow Fragment is able to fill in the protruding strand using its 5' → 3' polymerase activity. In an alternative embodiment, DSB end blunting by removal of 3' overhangs or fill-in of 5' overhangs can also be achieved using T4 DNA polymerase. In yet another embodiment, Mung Bean Nuclease may be used to catalyze the removal of single-stranded overhangs in DNA DSBs.

However, some genome-editing nucleases or restriction enzymes induce blunt-ended DSB. If such nucleases or restriction enzymes are used, the additional blunting step may be omitted.

Optionally, the DSB ends may be subjected to A-tailing to enable a T-A ligation with the first linker. In the context of the present invention, the term "tailing" refers to an enzymatic method for adding a non-templated nucleotide to the 3' end of a blunt, double-stranded DNA molecule. Correspondingly, "A-tailing" or "dA-tailing" means that a deoxyadenosine nucleotide is enzymatically added to the 3' end of the blunt DSB end, using, e.g., a Taq polymerase or a DNA Polymerase I Large (Klenow) Fragment that lacks both 5' →- 3' and 3' →- 5' exonuclease activity. Preferably, A-tailing is performed after blunting of the DSB ends. In one embodiment, the two reactions of DNA DSB end blunting and A-tailing are performed in two discrete method steps using the above-mentioned enzymes. In this case, an additional clean-up step may be required between the blunting and A-tailing steps. However, in a preferred embodiment, DNA DSB end blunting and A-tailing are performed in a single reaction step using, e.g., the NEBNext^{®} Ultra^{™} II End Repair/dA-Tailing Module, CD End Repair/dA-Tailing Module for Illumina (CD genomics), or a comparable enzyme kit. The skilled person will be familiar with additional enzymes that may be used to process DSBs ends prior to ligation of the first linkers. He or she will also be able to determine the reaction conditions suitable for blunting and A-tailing of DSB ends. When performing both reactions in a single method step using the NEB-Next^{®} Ultra^{™} II End Repair/dA-Tailing Module, the reactions may for instance be allowed to take place at a temperature of 20°C for a total of 30 minutes, followed by 65°C for 30 minutes. In the context of the invention the ends of the nucleic acid at a DSB, i.e., the DSB ends, are still considered DSB ends if they are modified by blunting and/or A-tailing.

The first linker that is ligated to the DNA DSB ends comprises a unique molecular identifier (UMI) sequence, and, optionally, a partial sequencing adapter region comprising a first sequencing primer binding site and/or a DNA sequence for attachment of a first full-length sequencing adapter, preferably, all of these.

In the context of the present invention, the first linker is also referred to as BreakTag-linker. The first linker is a double-stranded DNA molecule consisting of a first DNA strand (sense DNA strand) and a complementary or mostly complementary second DNA strand (antisense DNA strand). It may have a length of 25 to 100 base pairs (bp), preferably 35 to 85 bp, more preferably 45 to 75 bp and most preferably 50 to 70 bp, i.e., 50, 51, 52, 53, 54, 55, 56, 57, 58, 59, 60, 61, 62, 63, 64, 65, 66, 67, 68, 69, or 70 bp. In a particular preferred embodiment, the first linker has a length of 55-65 bp, i.e., of 55, 56, 57, 58, 59, 60, 61, 62, 63, 64, or 65 bp. In some embodiments, the first linker may also have a length greater than 100 bp, e.g., it may have a length of more than 100 bp, more than 110 bp, more than 120 bp, more than 130 bp, more than 140 bp or even more than 150 bp. Alternatively, the first linker may also be shorter than 25 bp. For instance, it may have a length of 24, 23, 22, 21, 20, 19, 18, 17, 16 or 15 bp.

The first linker comprises a unique molecular identifier (UMI). A UMI is a randomly assembled short nucleotide sequence that serves as a unique molecular tag. The UMI of the first linker labels a particular DSB after ligation and thus facilitates reliable identification of each DSB while effectively reducing errors and quantitative bias introduced during later amplification steps. The UMI may have a length of 1 to 20 bp, preferably of 3 to 15 bp and more preferably of 5 to 10 bp, i.e., of 5, 6, 7, 8, 9, or 10 bp. In a particular preferred embodiment, the first linker comprises a UMI with a length of 8 bp.

The first linker typically comprises a partial sequencing adapter region comprising a nucleic acid sequence that serves as a first sequencing primer binding site. During sequencing by synthesis, a first sequencing primer binds to the first sequencing primer binding site and serves as the starting point for the step-wise synthesis of a complementary DNA strand that is simultaneously sequenced. In case Illumina-based sequencing technologies are used for sequencing, the first sequencing primer binding site is also known as Read1 primer binding site. The sequencing primer binding site of the first linker may have a length of 15 to 25 bp, e.g., of 15, 16, 17, 18, 19, 20, 21, 22, 23, 24 or 25 bp.

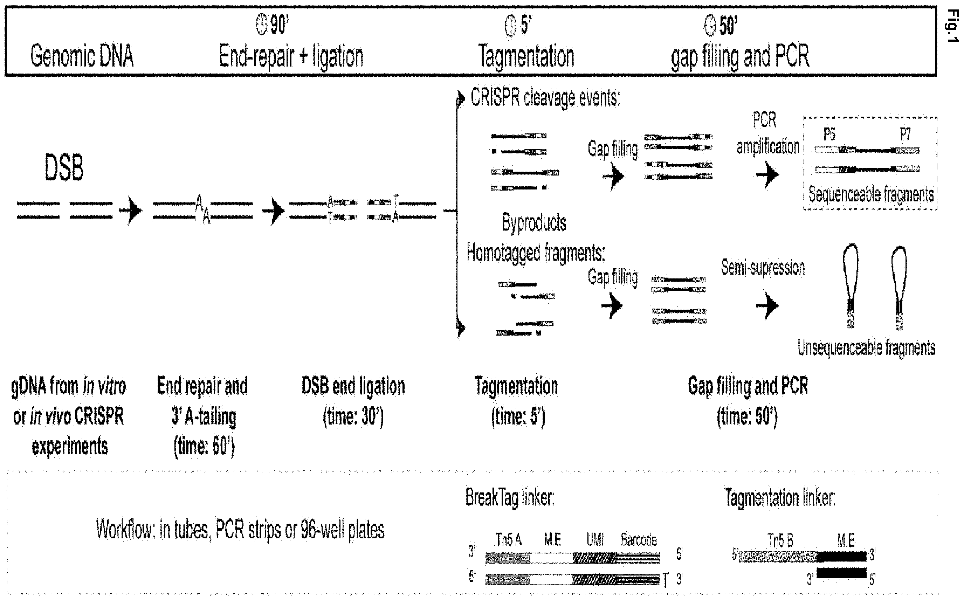

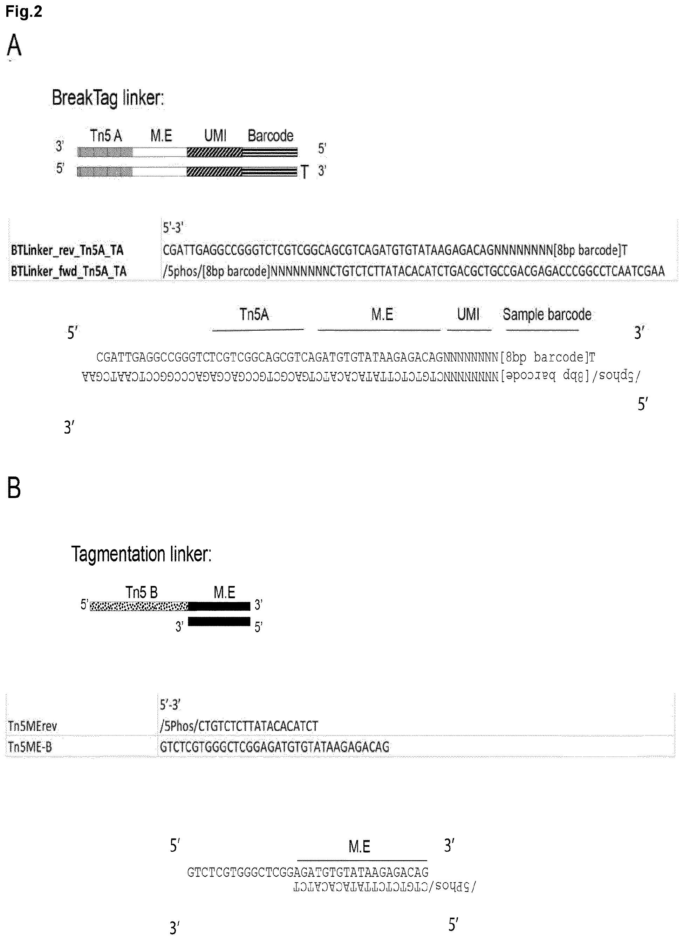

The partial sequencing adapter region of the first linker further comprises a nucleic acid sequence that serves as a binding site for the hybridization of a PCR primer required for attaching a first full-length sequencing adapter to the first linker during step f) of the method of the invention. In the context of the invention, this nucleic acid sequence will be referred to as first PCR handle. In Fig. 1 and in Fig. 2A, the first PCR handle is also referred to as Tn5A site. In the context of the invention, the term "full-length sequencing adapter" refers to an oligonucleotide sequence that facilitates clonal amplification and sequencing reactions. Preferably, the sequencing is done using well-established Illumina-based sequencing platforms, so that the first full-length sequencing adapter which is attached to the first linker during step f) comprises a p5 or a p7 flow cell binding site and, optionally, an i5 or i7 index sequence. Dependent on whether it comprises the p5 or p7 flow cell binding site, the full-length sequencing adapter is referred to as p5 or p7 sequencing adapter, respectively, in the context of the invention. The p5 and p7 flow cell binding sequences are required for a sequencing library to bind and generate clusters on an Illumina flow cell, i.e., to anneal the DNA fragments that are to be sequenced to complimentary p5 and p7 oligos firmly grafted to the surface of an Illumina flow cell. These flow cell oligos later-on act as primers for the synthesis of strand complimentary to the fragment that is to be sequenced. As the name implies, the optional p5/p7 index sequence allows the skilled person to perform multiplexing, i.e. to pool several libraries from different samples in a combined sequencing run. During analysis of the sequencing results, the specific index sequences enable the allocation of the different sequencing reads to their corresponding samples. The p5 sequencing adapter used in the context of the invention typically comprises an i5 index site and the p7 sequencing adapter used in the invention typically comprises an i7 index site.

Preferably, the first PCR handle serves as the binding site for a p5 sequencing adapter. However, alternatively, the first PCR handle may also serve as the binding site for a p7 sequencing adapter. The first PCR handle usually has a length of 10 to 20 bp, i.e., of 10, 11, 12, 13, 14, 15, 16, 17, 18, 19 or 20 bp. In a preferred embodiment the first PCR handle has a length of 13 to 16 bp, e.g., 13, 14, 15, or 16 bp.

In a preferred embodiment, the sense strand of the first PCR handle of the first linker has a nucleic acid of 5'-TCGTCGGCAGCGTC-3' (SEQ ID NO: 1). Preferably, the first PCR handle or parts thereof may also form at least a part of the first sequencing primer binding site of the first linker. For instance, in case Illumina-based sequencing platforms are used, the first PCR handle or parts thereof may form a part of the Read1 primer binding site.

The partial sequencing adapter region of the sense strand of the first linker that comprises the first sequencing primer binding site and the first PCR handle may have a nucleic acid sequence of 5'-TCGTCGGCAGCGTCAGATGTGTATAAGAGACAG-3' (SEQ ID NO: 2). SEQ ID NO: 2 corresponds to the nucleic acid sequence of the commercially available Read 1-Nextera Transposase Adapter.

The design of the first linker can be easily modified and adapted to other suitable sequencing technologies that are not Illumina-based. For instance, in an alternative embodiment the first PCR handle present in the first linker may serve as a binding site for a first sequencing adapter suitable for Ion Torrent^{™}-sequencing.

In case a blunt DSB end is not subjected to A-tailing prior to ligation of the first linker, the first linker needs to have a blunt end as well, so that the blunt end of the first linker can be directly ligated to the blunt DSB end.

However, if the DSB ends of the DNA have been subjected to A-tailing, the first linker furthermore comprises a single deoxythymidine triphosphate overhang at the 3' end to facilitate ligation with the 3' A-overhang at the processed DSB end and to prevent formation of first linker dimers. Optionally, a more stable phosphorothioate linkage (instead of phosphodiester) can be used to attach the 3'T to the first linker. The 5' end of the first linker that is to be ligated to the DSB end preferably carries a 5' phosphate group. The first linker is to be ligated to the DSB end in an orientation so that the UMI is located adjacent to the (processed) DSB end. Accordingly, the partial sequencing adapter of the first linker forms the free end or is located near to the free end of the first linker-DNA conjugate.

In an optional embodiment, the first linker further comprises at least one (e.g., at least two) identifying barcode regions. In the context of the present invention, the barcode region may also be referred to simply as barcode or as sample barcode. A barcode differs from a UMI in that it does not uniquely identify a single DSB, but rather labels all DSBs present in a DNA of a given sample. A barcode sequence thus allows the combination of different samples in a single sequencing run, as all sequencing reads can be assigned to their respective samples. When combined with Illumina i5 or i7 indexes, the additional barcode allows for an extra layer of indexing. The barcode may have a length of 1 to 25 bp, preferably 3 to 20 and most preferably 5 to 15 bp, e.g., 5, 6, 7, 8, 9, 10, 11, 12, 13, 14 or 15 bp. In a particularly preferred embodiment, the barcode has a length of 8 bp. In embodiments where the first linker comprises a barcode in addition to the UMI, the barcode may be arranged to be located between the UMI and the DSB end after ligation. Alternatively, the barcode may also be located between the UMI and the partial sequencing adapter region.

The first linker may further comprise additional DNA sequences. For instance, an additional DNA sequence that assists in the attachment of the full-length sequencing adapter may be located at the end that is to be free after the first linker has been ligated to the DSB end. This additional DNA sequence may exhibit a single-stranded DNA overhang, such as, e.g., a poly-A tail.

In a preferred embodiment, the first linker is a double-stranded DNA molecule consisting of a sense DNA strand comprising a nucleic acid sequence having at least 50% sequence identity to SEQ ID NO: 10 and a complementary antisense DNA strand comprising a nucleic acid sequence having at least 50% sequence identity to SEQ ID NO: 11. The sense DNA strand of the first linker may also comprise a nucleic acid sequence having at least 55, at least 60, at least 65, at least 70, at least 75, at least 80, at least 85, at least 90, at least 91, at least 92, at least 93, at least 94, at least 95, at least 96, at least 97, at least 98, or at least 99% sequence identity to SEQ ID NO: 10. In one embodiment, the sense DNA strand of the first linker may also comprise a nucleic acid sequence having 100% sequence identity to SEQ ID NO: 10. It may also consist of SEQ ID NO: 10. The antisense strand of the first linker may also comprise a nucleic acid sequence having at least 55, at least 60, at least 65, at least 70, at least 75, at least 80, at least 85, at least 90, at least 91, at least 92, at least 93, at least 94, at least 95, at least 96, at least 97, at least 98, or at least 99% sequence identity to SEQ ID NO: 11. In one embodiment, the antisense DNA strand of the first linker may also comprise a nucleic acid sequence having 100% sequence identity to SEQ ID NO: 11. It may also consist of SEQ ID NO: 11.

In a particular preferred embodiment, the first linker is a double-stranded DNA molecule consisting of a sense DNA strand comprising a nucleic acid sequence having at least 50% sequence identity to SEQ ID NO: 3 and a complementary antisense DNA strand comprising a nucleic acid sequence having at least 50% sequence identity to SEQ ID NO: 4. The sense DNA strand of the first linker may also comprise a nucleic acid sequence having at least 55, at least 60, at least 65, at least 70, at least 75, at least 80, at least 85, at least 90, at least 91, at least 92, at least 93, at least 94, at least 95, at least 96, at least 97, at least 98, or at least 99% sequence identity to SEQ ID NO: 3. In one embodiment, the sense DNA strand of the first linker may also comprise a nucleic acid sequence having 100% sequence identity to SEQ ID NO: 3. It may also consist of SEQ ID NO: 3. The antisense strand of the first linker may also comprise a nucleic acid sequence having at least 55, at least 60, at least 65, at least 70, at least 75, at least 80, at least 85, at least 90, at least 91, at least 92, at least 93, at least 94, at least 95, at least 96, at least 97, at least 98, or at least 99% sequence identity to SEQ ID NO: 4. In one embodiment, the antisense DNA strand of the first linker may also comprise a nucleic acid sequence having 100% sequence identity to SEQ ID NO: 4. It may also consist of SEQ ID NO: 4.

The skilled person will be aware of the conditions and enzymes required for ligating the first linker to the DSB end. For instance, the first linker can be ligated to the DSB end using, e.g., a T4 DNA Ligase or the NEBNext^{®} Ultra^{™} II Ligation Module in a ligation reaction that lasts for 15 min to 60 min, e.g., for 20, 30, 40 or 50 min, preferably for 15 min at 20°C to 25 °C, preferably at 20°C, followed by 15 min at 37°C. The first linker is added to the ligation reaction at a concentration of 50-300 nM, e.g., 50, 75, 100, 125, 150, 175, 200, 225, 250, 275, or 300 nM. Preferably, the first linker is added at a concentration of 50 to 150 nM e.g., 50, 60, 70, 80, 90, 100, 110, 120, 130, 130, 140, or 150 nM. In a particularly preferred embodiment, the first linker is added at a concentration of 100nM.

In a preferred step c) of the method according to the invention, non-ligated first linkers may be removed so as to not interfere with sequencing. First linkers that did not ligate to DSB ends are preferably removed by size selection using, e.g., Solid Phase Reversible Immobilization (SPRI) magnetic beads such as those provided by the Agencourt^{®} AMPure^{®} XP-kit. These magnetic beads are coated with carboxyl molecules that can reversibly bind to DNA fragments of a certain minimum size in the presence of polyethylene glycol (PEG) and salt. In contrast, contaminants such as non-bound first linkers are rinsed away. This step is preferably done at 20 to 28°C, for 5 to 60 min. In a preferred embodiment removal of non-ligated first linkers using the above-described magnetic beads will be allowed to take place for 5 min at a temperature of 23°C. In an alternative embodiment, gel size exclusion and/or silica column purification may be used to exclude non-ligated linkers from the ensuing method steps.

In step d) of the method according to the invention, the first linker-DNA conjugates obtained in step b) are enzymatically fragmented and the different fragments are tagged with a second linker. Fragmenting and tagging of the first linker-DNA conjugates is achieved in a single, so-called "tagmentation" step using a transposase, such as, e.g., Tn5 transposase or MuA transposase. In a preferred embodiment, the transposase is a hyperactive transposase, such as a hyperactive Tn5 transposase comprising E54K and L372P point mutations. During tagmentation, the transposase cleaves the DNA and adds the second linker to the 5' end of each DNA strand. This reaction may occur in solution or by using bead-linked transposomes, which may allow for more uniform tagmentation.

The second linker typically comprises a double-stranded transposase recognition motif and a single-stranded overhang. The overhang is a 5' overhang and serves as a handle for the addition of a second sequencing adapter.

The second linker may comprise a transposase recognition motif, and a partial sequencing adapter comprising a second sequencing primer binding site and a sequence suitable for attachment of a second full-length sequencing adapter.

In a preferred embodiment, the transposase recognition motif of the second linker is a Tn5 transposase recognition motif. The wild type Tn5 transposon is normally flanked by two nearly identical inverted insertion sequences (IS50L and IS50R), each containing two inverted 19 bp end sequences known as outside end (OE) and inside end (IE). It was however found in the past that these wild type end sequences exhibit rather low activity, which led to their replacement by a 19 bp hyperactive derivative (mosaic end, ME). Accordingly, the transposase recognition motif of the second linker is, preferably, a double-stranded mosaic element (ME) of 19 bp length having a nucleic acid sequence of 5'-CTGTCTCTTATA-CACATCT-3' (SEQ ID NO: 5) that is recognized by Tn5. The ME of the second linker also forms a first part of a second sequencing primer binding site. In the case Illumina-based sequencing platforms are to be used for sequencing, this second sequencing primer binding site is referred to as Read2 primer binding site.

Of note, the transposase recognition motif of the second primer binding site does not necessarily have to be a Tn5 ME. It may, e.g., also be formed of the wild type Tn5 end sequences OE/IE. The transposase recognition motif may also be a modified Tn5 recognition sequence that differs from the ME of SEQ ID NO: 5. It may, e.g., be a Tn5 recognition sequence having only 99, 98, 97, 96, 95, 94, 93, 92, 91, 90, 85, 80, 75, 70 65, 60, 55, 50, 45, 40, 35, 30, 25, 20, 15, or 10% sequence identity to SEQ ID NO:5.

In another embodiment, the transposase recognition site may also be or comprise the recognition sites of MuA transposase or a modified version thereof. For instance, the transposase recognition site may also be or comprise one or more of the recognition sites L1, L2 and L3 or R1, R2 and R3 of MuA transposase or a modified version thereof.

In addition to the double-stranded transposase binding motif, the second linker further comprises a single-stranded 5' overhang. This single-stranded overhang may have a length of 10 to 20 nucleotides (nt), i.e., of 10, 11, 12, 13, 14, 15, 16, 17, 18, 19 or 20 nt. In a preferred embodiment, the overhang has a length of 15 nt. The overhang of the second linker comprises a partial sequencing adapter comprising a nucleic acid sequence which provides the second part of the second sequencing primer binding site (e.g., the Read2 primer binding site). In addition, this nucleic acid sequence serves as a binding site for the hybridization of a PCR primer required for attaching a second full-length sequencing adapter to the second linker in step f) of the method of the invention. Accordingly, in the context of the invention, this nucleic acid sequence will be referred to as second PCR handle. In Fig. 1 and Fig. 2A, the second PCR handle is also referred to as Tn5B site. When Illumina-based technologies are to be used for sequencing, the second full-length sequencing adapter that binds to the second PCR handle is either a p5 or a p7 sequencing adapter, as described herein. The second PCR handle of the second linker usually has a length of 10 to 20 nt, i.e., of 10, 11, 12, 13, 14, 15, 16, 17, 18, 19 or 20 nt. In a preferred embodiment the second PCR handle has a length of 13 to 16 nt, e.g., 13, 14, 15, or 16 nt. The second PCR handle of the second linker may have a nucleic acid sequence of 5'-GTCTCGTGGGCTCGG-3' (SEQ ID NO: 6).

When the first linker comprises a first PCR handle having a nucleic acid sequence suitable for the attachment of the p5 sequencing adapter, the second linker comprises a second PCR handle having a nucleic acid sequence suitable for the attachment of a p7 sequencing adapter. Conversely, the second linker comprises a second PCR handle having a nucleic acid sequence suitable for the attachment of the p5 sequencing adapter in case the first linker comprises a first PCR handle having a nucleic acid sequence suitable for the attachment of a p7 sequencing adapter.

In a preferred embodiment of the invention, the first PCR handle of the first linker comprises a nucleic sequence suitable for attachment of the p5 sequencing adapter and the second PCR handle of the second linker comprises a nucleic acid sequence suitable for attachment of the p7 sequencing adapter.

As with the first linker, a person skilled in the art will be aware of how to modify and adapt the second linker design to other suitable sequencing technologies that are not Illumina-based. For instance, in an alternative embodiment, the second PCR handle present in the second linker may comprise a nucleic acid sequence suitable for the attachment of a sequencing adapter for Ion Torrent^{™}-sequencing. It is, as will become clear from the following explanations, however an essential feature of the invention that the first and second linkers must differ in their nucleic acid sequence, so that they are unable to hybridize with each other.

In a preferred embodiment, the second linker comprises a sense nucleic acid sequence having at least 50% sequence identity to 5'-GTCTCGTGGGCTCGGAGATGTGTATAA-GAGACAG-3' (SEQ ID NO: 7). Alternatively, the second linker may also comprise a sense nucleic acid sequence having at least 55, at least 60, at least 65, at least 70, at least 75, at least 80, at least 85, at least 90, at least 91, at least 92, at least 93, at least 94, at least 95, at least 96, at least 97, at least 98, or at least 99% sequence identity to SEQ ID NO: 7. In one embodiment, the second linker may also comprise a sense nucleic acid sequence having 100% sequence identity to SEQ ID NO: 7. Preferably, the second linker has a sense strand consisting of SEQ ID NO: 7 and corresponds to a commercially available Read 2 Nextera Transposase adapter.

Transposase-mediated tagmentation is a well-established method for the preparation of sequencing libraries. The skilled person will therefore be able to determine suitable conditions for performing the reaction. For instance, when using Tn5, fragmenting and tagmenting of the first linker-DNA conjugates will take 1 to 15 min, at a temperature of 37 to 70 °C. In a preferred embodiment, the tagmentation reaction is allowed to take place for 5 min at 55°C in a solution containing 40mM Tris-CI pH 7.5, 40mM MgCl2 in H2O and 25% dimethylformamide with Tn5 at 1.25 ng/µL.

Tagmentation of the double-stranded DNA fragments using a Tn5 transposase results in a 9-nt gap between the non-transferred strand and the target DNA of the transposase. Similarly, tagmentation using MuA transposase will result in a 5-nt gap. These gaps in the tagged double-stranded DNA fragments need to be filled by polymerase-chain-reaction (PCR) in step e) of the method according to the invention. For this, the PCR comprises a reaction step at which the gaps introduced by the transposase are filled in by a DNA polymerase, e.g., at 72°C for 1 to 30 min, preferably for 5 to 20 min, or 10 to 15 min, most preferably for 5 min. Upon completion, the PCR will result in two distinct populations of DNA fragments. The first population consists of double-stranded DNA fragments that are flanked by first linkers on one side and second linkers on the other side (heterotagged fragments). In contrast, the second population consists of double-stranded DNA fragments that are flanked only by second linkers on both sides and do not comprise the first linker (homotagged fragments).

After completion of the gap-filling PCR during step e), the method of the invention comprises a step during which the heterotagged population of DNA fragments is enriched for further sequencing library preparation. In the context of the invention, the terms "to enrich" and "enriching" are intended to mean that the percentage of heterotagged fragments inside the sample is selectively increased by amplifying only the heterotagged fragments and/or removing homotagged fragments from the sample. In some embodiments, "enriching" may be understood to mean "isolating", i.e., to end up with a sample that comprises only, or virtually only heterotagged fragments and no or virtually no homotagged fragments. Enrichment of the heterotagged population of DNA fragments can be achieved by another PCR amplification step. After denaturation of the double-stranded homo- and heterotagged DNA fragments, a fraction of the now single-stranded homotagged DNA fragments that comprise only the second linker at both ends will form intramolecular hairpins due to complementary base-pairing between the two second linkers at both ends of the same strand. Consequently, these hairpins are excluded from amplification during PCR. In contrast, the first and second linkers flanking each strand of the heterotagged DNA fragments will not hybridize with each other and thus will not form intramolecular hairpins. Accordingly, only the heterotagged fragments should be exponentially PCR amplifiable. However, homotagged DNA fragments having a size of more than 1 kb often do not form intramolecular hairpins, so that, in principle, they can still be PCR amplified. It may therefore be necessary to exclude these larger homotagged fragments by means of size selection to successfully isolate only the PCR-amplified heterotagged DNA fragments, which will usually have an average length of only about 250 to 600 nt. Suitable methods for size selecting DNA fragments are known to the person skilled in the art. For instance, size selection of the heterotagged fragments may be achieved by separation after gel-electrophoresis or size selection using SPRI.

Accordingly, in a preferred embodiment, the heterotagged DNA fragments are enriched by a combination of PCR amplification to exclude hairpin-forming homotagged DNA fragments of usually less than about 1 kb, followed by size selection to exclude larger amplified homotagged DNA fragments with a length of usually about 1 kb or more. However, in another embodiment, enrichment of the heterotagged DNA fragments may also be done by PCR amplification only, i.e., without a subsequent size selection step.

In the course of PCR amplification to enrich the heterotagged fragments, the full-length first and second sequencing adapters are also attached to the heterotagged fragments. For this purpose, primers that comprise the full-length adapter sequences and that are capable of hybridizing to the first and second PCR handles of the partial sequencing adapter regions of the first and second linkers at both ends of the fragments are used. In case Illumina-based sequencing technologies are to be used, the full-length sequencing adapters added to the first linker and the second linker flanking the DNA fragments of the heterotagged population comprise an Illumina flow cell binding sequence. In more detail, the full-length sequencing adapter comprising the p5 flow cell binding site is preferably attached to the first PCR handle of the heterotagged fragment, whereas the full-length sequencing adapter comprising the p7 flow cell binding site is attached to the second PCR handle of the heterotagged fragment. In a preferred embodiment in which Illumina-based sequencing technologies are used for sequencing, the first primer used for amplifying the heterotagged fragments and adding the first full-length p5 sequencing adapter has a nucleic acid sequence comprising SEQ ID NO: 8 or consist thereof and the second primer used for amplifying the heterotagged fragments and adding the second full-length p7 sequencing adapter has a nucleic acid sequence comprising SEQ ID NO: 9 or consists thereof.

Accordingly, in a preferred embodiment, at the end of the PCR amplification in step f) of the method according to the invention, a sequencing library is obtained consisting or predominantly consisting of heterotagged DNA fragments flanked by a full-length p5 sequencing adapter on one end and a full length p7 sequencing adapter at the opposite end that can be hybridized to an Illumina flow cell for subsequent sequencing. Obviously, in case sequencing platforms are to be used that are not Illumina-based, the sequencing library will consist or predominantly consist of fragments flanked by different types of adapters such as, e.g., adapters required for Ion Torrent^{™}-sequencing. The skilled person will have no problem to adapt the method according to the invention to such alternative sequencing methods.

Prior to sequencing, the quality of the sequencing library is preferably controlled using e.g., a Bioanalyzer. This step allows for verification of the fragment sizes and the detection of possible contaminations in the sample.

As explained herein, sequencing of the thus obtained sequencing library is preferably done using an Illumina-based sequencing technology/platform that is well known to the person skilled in the art, e.g., HiSeq, NextSeq or NovaSeq. However, as explained above, other sequencing technologies may be used instead, including, e.g., Ion Torrent^{™}-sequencing by slightly adapting the design of the first and second linkers used.

In a preferred embodiment, the sequencing library is subjected to paired-end sequencing. During paired-end sequencing, both ends of a given DNA fragment are sequenced, i.e., a given DNA fragment is first read and sequenced in one direction from one end before this process is repeated in the opposite direction from the other end. In the case of Illumina sequencing, paired-end sequencing starts from the Read1 primer bound to the Read1 primer binding site on one side of a DNA fragment and proceeds in the 5'→-3' direction along the forward strand. The resulting sequencing read is referred to as read 1. The second read, which is herein referred to as read 2, starts at the Read2 primer bound to the Read2 primer binding site at the other side of the DNA fragment and extends in 5'→-3' direction along the reverse strand. Since paired-end sequencing produces twice the number of sequencing reads than single-read sequencing, it enables more accurate read alignment to a reference DNA. Of course, in some embodiments, only single-read sequencing may be performed, i.e., each fragment in the sequencing library is sequenced only in a single direction. Accordingly, if Illumina-based sequencing is used, only one of read 1 or read 2 is obtained for each fragment during sequencing.

The sequencing of the sequencing library will result in a plurality of sequencing reads that can be bioinformatically analyzed to detect the DNA DSBs in step h) of the method according to the invention. For this analysis, a software pipeline suitable for mapping the obtained sequencing reads to a reference DNA and detecting and, optionally, quantifying the total amount of DSBs in the original DNA can be used.

To detect a DSB in a DNA of a sample and/or identify its location, the bioinformatic analysis during step h) of the method of the invention comprises at least the steps of
i) filtering for sequencing reads that contain the UMI and, optionally, the sample barcode;
ii) mapping the filtered sequencing reads to a reference DNA;
iii) filtering out sequencing reads mapping to multiple positions of the reference DNA, or representing low quality alignments;
iv) identifying the UMI labelling an individual DSB.

To quantify the total amount of DSBs in a DNA of a sample, the bioinformatic analysis performed during step h) of the method of the invention comprises at least the above-mentioned steps to detect a DSB in a DNA of a sample followed by counting all reads with different UMI.

In a particularly preferred embodiment, step h) of the method comprises identifying on- and/or off-targets of a genome-editing nuclease selected from the group comprising a CRISPR nuclease, a Meganuclease, a Zinc Finger Nuclease (ZNF), or a transcription activator-like effector nuclease (TALEN), preferably, of a CRISPR nuclease. In the context of the invention, the term "on-target" refers to an intended and specific DNA DSB induced by the genome-editing nuclease at the exact site within the DNA for which the genome-editing nuclease has been programmed, e.g., by designing a gRNA that targets a CRISPR nuclease to a specific DNA site of interest. Correspondingly, the term "off-target" is to be understood as a DSB induced by the genome-editing nuclease outside the desired target sequence, i.e. it refers to an unintended DNA DSB generated by the genome-editing nuclease in regions of the DNA that exhibit a similar sequence to the intended target site, thereby causing an undesired mutation.

If the method of the invention is used for identifying on- and/or off-targets of a genome-editing nuclease as described herein, bioinformatic analysis during step h) comprises at least steps of
i) filtering for sequencing reads that contain a UMI and, optionally, the sample barcode;
ii) aligning the filtered sequencing reads to a reference DNA and counting the number of reads representing individual DSBs based on their corresponding UMI and, optionally, sample barcode;
iii) identifying candidate loci for being edited by the genome-editing nuclease;
iv) obtaining the sequence context of the candidate loci and focusing only on candidate loci exhibiting a region with a desired minimum sequence identity to the intended target site of the genome-editing nuclease;
v) counting the number of reads representing DSBs within said region and identifying the cleavage site; and
vi) test if the enrichment of reads seen in the sequencing library obtained from DNA targeted with the genome-editing nuclease is significant compared to a control library obtained from DNA that has not been targeted with a genome-editing nuclease.

In a particular preferred embodiment, the genome-editing nuclease is a CRISPR nuclease and the bioinformatic analysis during step h) of the method according to the invention comprises identifying the on- and/or off-targets of said CRISPR nuclease. Accordingly, step h) of the method comprises at least the steps of
i) filtering for sequencing reads that contain a UMI and, optionally, a sample barcode;
ii) aligning the filtered sequencing reads to a reference DNA and counting the number of reads representing individual DSBs based on their corresponding UMI and, optionally, sample barcode;
iii) identifying candidate loci for being CRISPR-edited;
iv) obtaining the sequence context of the candidate loci and keeping only candidate loci comprising a PAM sequence and having a protospacer region exhibiting a desired minimum sequence identity to the sgRNA sequence used to guide the CRISPR nuclease;
v) counting the number of reads representing DSBs within the protospacer region and identifying the cleavage site; and
vi) testing if the enrichment of reads seen in the sequencing library obtained from DNA targeted with a CRISPR nuclease is significant compared to a control library obtained from DNA that has not been targeted with a CRISPR nuclease.

This analysis can be done by any suitable bioinformatic analysis pipeline capable of carrying-out the above-mentioned analysis steps. For example, the present inventors developed a two-step analysis pipeline capable of performing the above-mentioned steps on sequencing reads obtained after Illumina-based sequencing. The first step of this workflow involves the use of a specifically designed pipeline termed by the inventors "Breaktag NGSpipe2go pipeline".

For step i) of the analysis, the Breaktag NGSpipe2go pipeline scans for sequencing reads (single- or paired-end) containing the expected UMI and/or, optionally, the sample barcode in the 5' end of read 1. This is achieved by using a program which matches a supplied Perl regular expression in read 1, and outputs only those reads matching such pattern. A person skilled in the art knows that the term "regular expression" refers to strings with a very particular syntax. These strings are called "patterns" and are used to determine if some other string, called the "target", has (or doesn't have) the characteristics specified by the pattern in a process known as "matching" the target string against the pattern.

During step ii) the resulting reads are mapped to the reference DNA, preferably a reference genome, using the well-established Burrows-Wheeler Aligner (BWA) software (Li, 2013) with a pre-determined seed length and default scoring/penalty values for mismatches, gaps and read clipping. In principle, mapping could work with any pre-determined seed length as long as it is set to a smaller value than the maximum read length output of the respective sequencer used for sequencing. Preferably, the seed length is however set to 10-30, more preferably, 15-25 or 18-20. In a particular preferred embodiment, the seed length is set to 19. Only reads with a pre-set mapping quality (defaults to 60) are retained to ensure low probability of the alignment/base being wrong and exclude reads mapping to multiple positions of the genome.

After step ii), an optional, final, filtering step may be performed in order to remove potentially duplicated reads stemming from PCR. For this, close spatial consecutive reads mapping within a window of 0 to 100 nucleotides, preferably 10 to 75 or 20 to 50 nucleotides, more preferably 25 to 40 nucleotides, most preferably 30 nucleotides and a UMI differing with up to 2 mismatches, i.e., 0, 1, or 2 mismatches are considered PCR duplicates and only one of the reads is kept. The resulting reads are aggregated per position and reported as a Browser Extensible Data (BED) file.

Using the software package developed by the present inventors, the next steps of the analysis are performed by the second part of the two-step pipeline, referred to by the inventors as "BreakinspectoR". BreakinspectoR is an R package which performs a guided search toward putative on-/off-targets of a CRISPR nuclease. As input, it takes: i) the BED files generated by the Breaktag NGSpipe2go pipeline of a pair of a library that has been produced by the method of the invention from DNA targeted by the CRISPR nuclease (target library) and a control library that was produced from DNA which was not targeted by the CRISPR nuclease (non-target library) , along with ii) the sgRNA sequence used to guide the CRISPR nuclease, iii) the PAM sequence, iv) the number of base-pairs upstream to the PAM where the CRISPR nuclease is expected to cleave the DNA, and v) the number of mismatches allowed in the sgRNA guide.

For step iii), BreakinspectoR scans for loci in the target library containing a minimum coverage due to the presence of stacks of read ends which makes them eligible to be targeted.

In step iv), the obtained regions are complemented with the genomic sequence context, and only those loci which are located at not more than a pre-defined distance (at most "N" nucleotides) upstream from a pre-set PAM and contain a protospacer region comprising not more than a predefined number of (up to "M") mismatches with regard to the gRNA guide are kept as candidates. The PAM is usually set to the canonical sequence "NGG", however, various other PAM sequences that are recognized by different CRISPR nucleases are known in the art. Accordingly, the skilled person will be able to pre-set the PAM to any one of these alternative sequences, dependent on which CRISPR nuclease is used to create the target library. The number of nucleotides "N" is preferably set by default to the canonically accepted value 3 for the Cas9 CRISPR nuclease, however, in alternative embodiments, "N" may also correspond to less than 3 such as, e.g. 1 or 2, or to more than 3, such as, e.g., 4, 5, 6, 7, 8, 9, or 10. "N" may even be a negative number such as -1, -2, -3, -4, or -5. The number of allowed mismatches with regard to the gRNA "M" is, by default, preferably set to 7. Again, the skilled person may alternatively set "M" to a value lower than 7, e.g., 6, 5, 4, 3, 2, or 1 or to a value higher than 7, such as, e.g., 8, 9, 10, 11, 12 or up to a value that corresponds to the total length of the sgRNA used. BreakinspectoR differentiates on-targets from off-targets by computing the number of mismatches between the provided sgRNA sequence used to guide the CRISPR nuclease and the genomic sequence context of the targeted locus, assuming on-targets only will contain exactly 0 (zero) mismatches.

Using the input BED files, BreakinspectoR next scouts the number of unique reads for the obtained loci of interest in the target and non-target libraries. In order to distinguish true edited sites in the target library and test if the enrichment of reads seen in the targeted library is significant compared to the non-targeted library, BreakinspectoR may perform a statistical test, e.g., a binomial test, to derive a p-value, using the number of breaks within the region in the target library as the number of trials, the total number of breaks in the whole target library as the total number of breaks in the whole target library, and the ratio between number of breaks within the region in the nontarget library and the total number of breaks in the whole nontarget library as the estimated success probability in each trial. In a preferred embodiment, BreakinspectoR may add a pseudocount to avoid dividing by 0. Q-values and local False Discovery Rate values are estimated for false-discovery rate (FDR) control, using well-established means, such as, e.g., the qvalue package (Storey et al., 2022).

Optionally, BreakinspectoR implements a complimentary method to estimate the false discovery of targets. For this, BreakinspectoR reshuffles the signal in the target library using several multinomially distributed random number vectors sampled with equal probabilities to the signal in the originally detected off-targets. Then, BreakinspectoR analysis is done in the reshuffled target library vs. the non-target library, and an FDR is estimated comparing the signal of each off-target called in the original target library to the targets detected in the reshuffled target library (where no targets are expected to be called).

The bioinformatic software used for analyzing the DSBs in a DNA preferably provides a clear and user-friendly visualization of the analysis results that allows the user to easily summarize the obtained data or use it for further analysis. For instance, the BreakinspectoR pipeline includes several handy visualizations to further analyze and summarize the on-/off-targets of a CRISPR nuclease detected. The visualized data may include, e.g., the analysis of fidelity of the sgRNA, sequence composition of target regions, frequency of mismatches per position of the protospacer, or the genomic distribution of the targeted regions.

Of note, the abovementioned two-step analysis workflow involving the use of the Breaktag NGSpipe2go and the BreakinspectoR pipelines may be easily adapted to analyze the on- and/or off-targets of other genome-editing nucleases such as Meganuclease, Zinc Finger Nuclease (ZNF), or transcription activator-like effector nuclease (TALEN). For this, instead of analyzing the obtained candidate loci suspected to be nuclease-edited for the presence of a PAM and comparing the sequence identity to a sgRNA, step iii) of the analysis comprises determining a predefined number of (up to "O") mismatches with regard to the defined DNA recognition sequence of the programmed genome-editing nuclease. It thus is sufficient to instruct BreakinspectoR that there is no PAM region, and to provide the distance to the end of the recognized motif where the nuclease is expected to cut.

Active Cas9 contains the two nuclease domains HNH and RuvC that cut the target and the non-target DNA strand, respectively, generating a DNA DSB (Jinek et al., 2012).

Early, seminal studies on CRISPR cutting properties described that both domains would always cut between the 3^{rd} and 4^{th} nucleotides ahead of the PAM sequence (van Overbeek et al., 2016; Allen et al., 2019), generating two blunt DNA ends that are religated then by the repair machinery, often generating a random indel at the cutsite (Jinek et al., 2012). Later studies revealed that a fraction of CRISPR repair outcomes is predictable and reproducible, suggesting that Cas9 DSB repair is not always random (Molla et al., 2020; van Overbeek et al., 2016; Allen et al., 2019; Shou et al., 2018; Chakrabarti et al., 2019). In parallel, other studies revealed that the RuvC domain, but not HNH, can cleave the non-target strand at positions 4, 5 or 6 ahead of the PAM, generating 1-3 nt-long 5' single-strand DNA (ssDNA) overhangs (Molla et al., 2020; Shi et al., 2019; Gisler et al., 2019). Upon repair, a blunt end may give rise to a random deletion, a template-independent insertion, or the initial sequence. In contrast, the generation of an overhang and DNA repair mediated by the NHEJ pathway can result in a predictable templated insertion, making it more desirable for precise, predictable genome-editing.

In the context of the invention, CRISPR nucleases that induced blunt-ended DSB are characterized as having a blunt scissile profile, whereas CRISPR nuclease that induce staggered/sticky DSB ends are characterized as having a staggered scissile profile.

The sequence determinants of scissile profile decision have not been elucidated due to the lack of tools that allow high-throughput assessment of DSB-end structure. There is an urgent need to elucidate sequence determinants of scissile profile, as this will allow better gRNA design for precise template-free CRISPR nuclease-mediated, preferably Cas9-mediated, gene editing.

The herein described method according to the invention has single-nucleotide resolution and usually comprises an end-repair step in which staggered DSB ends are enzymatically blunted prior to the ligation of the first linker. These features can be exploited during the bioinformatic analysis during step h) of the method in order to determine the scissile profile of a CRISPR nuclease, preferably of Cas9: Cas9-mediated DSBs contain two distinct sides or ends, one containing the PAM-sequence (PAM-proximal side) and the other containing the remaining protospacer sequence (PAM-distal side) As explained above, DSB end-blunting prior to ligation of the first linker involves the enzymatic chewing back, i.e., removal, of 3' ssDNA overhangs or the filling-in of 5' ssDNA overhangs. When analyzing the sequencing results during step h) of the method according to the invention, this removal of 3' ssDNA overhangs or the filling of 5' ssDNA manifests itself in the presence of nucleotide gaps or overlaps, respectively, between the first sequencing read which maps to the first strand of the reference DNA and starts from the PAM-proximal side (PAM-proximal read) of a given DSB and the first sequencing read mapping to the opposing strand of the reference DNA and starts from the PAM-distal site (PAM-distal read) of said DSB. When using Illumina-based sequencing, it is thus sufficient to determine at which nucleotide position read 1 starts at each side of a given DSB.

Because the canonical cleavage of Cas9 is the 3^{rd} nucleotide ahead of the PAM sequence, the length of the 3' or 5' overhangs of a staggered end of a given DSB can then be directly inferred from the size of the sequencing read (e.g., read 1) shift in relation to the expected cutsite.

Accordingly, the present invention also provides a method for determining whether a CRISPR nucleases coupled with a specific gRNA generates a DSB having blunted or a staggered ends, wherein the method comprises all steps a) to h) of the herein described method for detecting one or more DSBs in a DNA. Prior to step b) the method further comprises a processing step during which the DSB ends are enzymatically blunted, as described herein. To ultimately determine the scissile profile of a CRISPR nuclease, i.e., to identify whether a CRISPR nuclease generates a blunt or a staggered DNA DSB end, the analysis step h) of the method according to the present invention comprises at least the steps of
i) filtering for sequencing reads that contain a UMI and, optionally, a sample barcode;
ii) aligning the filtered sequencing reads to a reference DNA and counting the number of reads representing individual DSBs based on their corresponding UMI and, optionally, sample barcode, thereby differentiating between the reads that align on either strand of the reference DNA;
iii) identifying candidate loci for being CRISPR-edited;
iv) obtaining the sequence context of the candidate loci and keeping only candidate loci comprising a PAM sequence and having a protospacer region exhibiting a desired minimum sequence identity to the sgRNA sequence used to guide the CRISPR nuclease;
v) counting the number of reads representing DSBs within the protospacer region and identifying the cleavage site;
vi) testing if the enrichment of reads seen in the sequencing library obtained from DNA targeted with a CRISPR nuclease is significant compared to a control library obtained from DNA that has not been targeted with a CRISPR nuclease;
vii) identifying the presence of nucleotide gaps and/or overlaps, respectively, between sequencing reads starting from the PAM-proximal side and sequencing reads starting from the PAM distal-side of a given cleavage site to determine whether the CRISPR nuclease created a DSB having blunt or staggered break ends; and
viii) counting the number of PAM-proximal sequencing reads representing a staggered scissile profile, and testing if the enrichment of sequencing reads representing a staggered scissile profile seen in the sequencing library obtained from DNA targeted with a CRISPR nuclease is significant compared to a control library obtained from DNA that has not been targeted with a CRISPR nuclease.

Using this method, the inventors furthermore found that the scissile profile of a CRISPR nuclease was sequence-dependent, as the use of different sgRNAs resulted in strikingly distinct cut signatures. The method according to the invention may therefore also allow for the identification of optimal gRNAs for a given target region of interest in a DNA, which are capable of promoting the formation of staggered ends by a CRISPR nuclease, such as, e.g., Cas9.

The method according to the invention is suitable for a number of different uses. For instance, the method may be used for
(a) profiling the off-target activity of a genome-editing nuclease, preferably, a CRISPR nuclease;
(b) investigating repair kinetics of DSB breaks;
(c) assessing DNA DSB repair capacity by quantifying DSBs in a sample;
(d) detecting off-target activity of base editors;
(e) assessing specificity and fidelity of a CRISPR nuclease; and/or
(f) analysing the ratio of staggered to blunt-ended DSBs induced by a genome-editing nuclease, preferably, a CRISPR nuclease.

The use according to (a) requires a suitable bioinformatic analysis of the obtained sequencing results, which can be achieved, e.g., using the two-step analysis pipeline described herein.

The use of the method for investigating DSB repair kinetics according to (b) may be done in a cell or tissue *in vitro* or *in vivo* in an organism. In both cases, DNA DSBs are induced by any of the means described herein and DNA is isolated after various time points. The isolated DNA is subsequently subjected to the method according to the invention to detect the total number of DNA DSBs for each time point. The assessment of DNA DSB repair capacity by quantifying DSBs in a sample according to (c) is achieved by similar means. Here, the number of DNA DSBs detected after a given time period after DSB induction may be compared, e.g., to the number of DSBs in DNA of an identically treated cell, tissue or organism that has been isolated immediately after DSB induction. In addition, the total number of detected DSBs may also be compared to one or more reference values that are indicative of the number of DSBs after different time points, when repair of DNA DSBs is fully functional.

The method according to the invention may also be used to detect the off-target activity of base editors. Base editing refers to a modified form of the CRISPR/Cas system, which involves the introduction of single-nucleotide variants (SNVs) into DNA or RNA in living cells. In contrast to genome-editing via the conventional CRISPR/Cas system, base editing introduces targeted point mutations without inducing a double-strand break. In more detail, base editors are CRISPR nucleases / Cas proteins with one nuclease domain mutated independently of the other to create a DNA "nickase" capable of introducing a single-strand cut with the same specificity as a regular CRISPR/Cas9. The thus modified Cas proteins are further fused to a deaminase such as, e.g., APOBEC, that generates a base deamination at the non-target strand. At present, there a two main classes of base editors, so-called cytosine base editors (CBEs), which mediate a C to T change (or a G to A change on the opposite strand) of a DNA and adenine base editors (ABEs) that mediate an A to G change (or a T to C change on the opposite strand). For using the method according to the invention, the site-specific base-deamination induced by the deaminase domain of the modified Cas protein is removed *in vitro* using specific repair enzymes such as, e.g., EndoV (also known as deoxyinosine 3' endonuclease) from *Thermotoga maritima.* During this process an artificial DSB is generated that can be detected via the method of the invention.

As mentioned under (e), the method according to the invention may further be used for assessing the specificity and fidelity of a CRISPR nuclease. The CRISPR nuclease may be isolated from natural sources or may have been artificially designed. For instance, numerous engineered Cas9 and Cas12 nuclease variants are continuously developed in the hope to obtain a CRISPR nuclease with a notably reduced number of CRISPR off-targets and thus higher specificity. The scalability, precision and extremely fast workflow of the method according to the present invention are advantageous for assessing the target specificity of new variants in high-throughput manner.

Finally, the method according to the invention may be used for analysing whether a genome-editing nuclease, preferably a CRISPR nuclease, creates blunt-ended or sticky-ended DSBs as described herein. It is known that some CRISPR nucleases such as, e.g., Cas12a, are capable of inducing DSBs with sticky ends, i.e. DSB ends that exhibit a 4 to 5 nt single-stranded overhang. In contrast Cas9 is usually considered to induce DSB with blunt ends, although reports exist according to which Cas9 is able to induce DSB exhibiting a 1-3 nt overhangs. Whether a DSB exhibits sticky ends rather than blunt ends may affect repair of said DSB and may facilitate insertion of genes at the breakage sites. The method according to the invention can even analyse the ratio of staggered to blunt-ended DSBs.

The present invention further provides a kit that may be used for performing the methods according to the invention. The kit comprises
a) enzymes suitable for blunting and A-tailing of DNA DSB ends;
b) an enzyme suitable for ligating the first linker to the DNA fragment;
c) a transposase capable of catalyzing tagmentation with the second linker;
d) a set of first linkers;
e) a set of second linkers;
f) nucleotides; and
g) buffers.

The enzymes suitable for blunting DNA DSB ends may be any of the enzymes described herein, e.g., the *E*. *coli* DNA Polymerase I Large (Klenow) Fragment lacking the 5' →- 3' exonuclease activity of intact DNA polymerase I, but retaining its 5' →- 3' polymerase and 3' →- 5' exonuclease as well as its strand displacement activities. Alternatively, the kit may comprise a T4 DNA polymerase or Mung Bean Nuclease as an enzyme for blunting DSB ends. The enzymes suitable for A-tailing of the blunted DSB ends may be a Taq polymerase or a DNA Polymerase I Large (Klenow) Fragment that lacks both 5' →- 3' and 3' →- 5' exonuclease activity. Preferably, DNA DSB end blunting and A-tailing are performed in a single reaction step so that the kit may comprise, e.g., the NEBNext^{®} Ultra^{™} II End Repair/dA-Tailing Module or a comparable commercially available enzyme module.

The enzyme suitable for ligating the first linker to the end of a DSB may be a e.g., a DNA Ligase or the NEB-Next Ultra II Ligation Module.

The transposases present in the kit according to the invention may be a Tn5 transposase or a MuA transposase. In a preferred embodiment, the transposase provided by the kit is a Tn5 transposase, preferably a hyperactive Tn5 transposase. In a particularly preferred embodiment, the hyperactive Tn5 transposase may comprise E54K and L372P point mutations.

The kit further provides a set of the first and second linkers described herein. The set of first linkers comprises different first linkers that are distinguishable from each other by their specific UMIs. Each set of first linkers comprises at least 5, at least 10, at least 20, at least 30, at least 40, at least 50, at least 100, at least 200, at least 500, at least 1000, at least 1500, at least 2000, at least 2500, at least 3000, at least 4000, at least 5000, or at least 10000 distinct first linkers that are distinguishable by their respective UMI. Moreover, each set comprises at least 5, at least 10, at least 20, at least 30, at least 40, at least 50, at least 100, at least 200, at least 500, at least 1000, at least 1500, at least 2000, at least 2500, at least 3000, at least 4000, at least 5000, or at least 10000, at least 50000 or at least 100000 identical copies of each individual first linker.

The set of second linkers comprises at least 5, at least 10, at least 20, at least 30, at least 40, at least 50, at least 100, at least 200, at least 500, at least 1000, at least 1500, at least 2000, at least 2500, at least 3000, at least 4000, at least 5000, or at least 10000, at least 50000 or at least 100000 copies of the same second linker.

The kit may comprise additional components that are useful for performing the method according to the invention. For instance, in an optional embodiment, the kit may provide the SPRI magnetic beads such as those provided by the Agencourt^{®} AMPure^{®} XP-kit, which may be used for the removal of unbound first linkers. The kit may, optionally, further provide primers required for the amplification of the tagged DNA fragments or for the addition of the full-length sequencing adapters.

The kit preferably comprises multiple distinct compartments. One of these compartments comprises the set of first linkers, whereas a second compartment comprises the set of second linkers. The different enzymes are preferably provided in separate compartments. Likewise, the necessary buffers and nucleotides required for different reactions are preferably provided in separate compartments. However, buffers and nucleotides required for PCR reactions may be provided in a common compartment. In a particular preferred embodiment, all enzymes, buffers and reagents that are required for a given reaction step of the method according to the invention are provided in a single compartment to allow the user an easy step-by-step use of the kit. The kit furthermore preferably comprises a manual that guides the user through the different methods steps and provides him or her with all necessary information for successfully preparing the sequencing libraries according to the method of the invention. Optionally the kit may also provide the user with a software suitable for performing the bioinformatic analysis of the present method, as described herein. The software may be provided via the Internet or by means of a data carrier such as a CD or a USB-stick. The kit may be stored at a temperature of 2-6°C for several days to several weeks, e.g., 1 day to 4 weeks, 1 day to 3 weeks, 1 day to 2 weeks, or 1 day to 7 days. Preferably, the kit is stored at 2-6°C for less than 2 weeks. For long-term storage, the kit or composition may be stored at -20 to -80°C for a period of time ranging from several weeks to several months prior to use, e.g., from 4 weeks to 24 months, from 2 months to 20 months, from 4 months to 16 months, from 8 months to 14 months, or for about 12 months. In some embodiments, the kit may be stored at -80°C for even longer periods prior to use, e.g., for 3 years, for 4 years, for 5 years, for 6 years, for 7 years, for 8 years, for 9 years, or even for up to 10 years.

Taken together, the present invention provides a novel method called BreakTag that has the ability to faithfully detect DSBs in a DNA. In particular, the method allows the detection of DSBs across an entire genome with nucleotide resolution in both cell-based assays and *in vitro* by using the same workflow. Unlike other known state-of-the-art methodologies such as DISCOVER-seq and TTISS-seq, the method according to the invention profiles DSBs directly and therefore does not rely on the mere analysis of indirect markers for DSBs such as the detection of recruited DNA repair factors or the initiation of DSB repair by specific repair pathways. The disclosed method is particularly suitable for profiling the on- and off-target activity of various genome-editing nucleases, in particular DSB-inducing CRISPR nucleases such as Cas9, Cas12 or any of their variants. By a simple protocol modification, the method can even be adapted to profile the off-target activity of the more recently developed base editors. The methodology is unusually fast and can be performed in parallel from limited amounts of DNA, by using off-the-shelf-reagents. It uses a PCR suppression step to enrich for DSBs via tagmentation. This allows for a fast, protocol that can be easily implemented in any laboratory. Finally, the BreakTag method described herein is highly cost efficient. The sample costs for BreakTag is currently 13.26 euros excluding sequencing (see Tab. 2), which is considerably cheaper than similar methods like sBLISS with a cost of approximately 50 euros excluding sequencing (Bouwman et al., 2020). The smaller price is possible due to small number of enzymatic reactions and therefore allows a bigger profit margin in case of commercialization.

**Table 2: BreakTag workflow costs as of 2021**

| **Reaction** | **Price per library (EUR)** |
|---|---|
| End-prep: | 4.35 |
| Ligation: | 6.61 |
| Clean-up post-ligation: | 1.36 |
| PCR- Ultra II Q5: | 0.43 |
| Tagmentation using in-house produced Tn5 transposase: | 0.0031 |
| Clean-up post PCR: | 0.51 |
| **Total per library:** | 13.26 |

Also disclosed is a computer program product comprising instructions which, when the program is executed by a computer, cause the computer to carry out step h) of the method of the invention, i.e., the bioinformatic analysis of the sequencing results to detect the one or more DSBs, e.g., steps h) i) to vi) as defined above. The computer program product may further comprise instructions which, when the program is executed by a computer, cause the computer to identify the location of the one or more DSBs in the DNA and/or quantify the amount of DSBs in the DNA, and/or identify on- and/or off-targets of a genome-editing nuclease selected from the group comprising a CRISPR nuclease, Meganuclease, Zinc Finger Nuclease (ZNF), or transcription activator-like effector nuclease (TALEN), preferably, of a CRISPR nuclease. In particular, the computer program product may comprise instructions which, when the program is executed by a computer, cause the computer to carry out step h) comprising
i) filtering for sequencing reads that contain a UMI and, optionally, a barcode;
ii) aligning the filtered sequencing reads to a reference DNA and counting the number of reads representing individual DSBs based on their corresponding UMI and, optionally, barcode;
iii) identifying candidate loci for being CRISPR-edited;
iv) obtaining the sequence context of the candidate loci and keeping only candidate loci comprising a PAM sequence and, optionally, having a protospacer region exhibiting a desired minimum sequence identity to the sgRNA sequence used to guide the CRISPR nuclease;
v) counting the number of reads representing DSBs within the protospacer region and identifying the cleavage site; and
vi) testing if the enrichment of reads seen in the sequencing library obtained from DNA targeted with a CRISPR nuclease is significant compared to a control library obtained from DNA that has not been targeted with a CRISPR nuclease.

In addition, the computer product may further comprise instructions which, when the program is executed by a computer, cause the computer to carry out an analysis of the scissile profile of a CRISPR nuclease, i.e., to identify whether a CRISPR nuclease generates a blunt or a staggered DNA DSB end. For this, the computer product may comprise instructions which, when the program is executed by a computer, cause the computer to carry out step h comprising
i) filtering for sequencing reads that contain a UMI and, optionally, a barcode;
ii) aligning the filtered sequencing reads to a reference DNA and counting the number of reads representing individual DSBs based on their corresponding UMI and, optionally, barcode, thereby differentiating between the reads that align on either strand of the reference DNA;
iii) identifying candidate loci for being CRISPR-edited;
iv) obtaining the sequence context of the candidate loci and keeping only candidate loci comprising a PAM sequence and having a protospacer region exhibiting a desired minimum sequence identity to the sgRNA sequence used to guide the CRISPR nuclease;
v) counting the number of reads representing DSBs within the protospacer region and identifying the cleavage site;
vi) test if the enrichment of reads seen in the sequencing library obtained from DNA targeted with a CRISPR nuclease is significant compared to a control library obtained from DNA that has not been targeted with a CRISPR nuclease;
vii) identifying the presence of nucleotide gaps and/or overlaps, respectively, between sequencing reads starting from the PAM-proximal side and sequencing reads starting from the PAM distal-side of a given cleavage site to determine whether the CRISPR nuclease created a DSB having blunt or staggered break ends; and
viii) counting the number of PAM-proximal sequencing reads representing a staggered scissile profile, and testing if the enrichment of sequencing reads representing a staggered scissile profile seen in the sequencing library obtained from DNA targeted with a CRISPR nuclease is significant compared to a control library obtained from DNA that has not been targeted with a CRISPR nuclease.

The computer program product may also comprise instructions which, when the program is executed by a computer, cause the computer to carry out any of the uses of the method described herein, i.e.,
(a) profiling the off-target activity of a genome-editing nuclease, preferably, a CRISPR nuclease;
(b) investigating repair kinetics of DSB breaks;
(c) assessing DNA DSB repair capacity by quantifying DSBs in a sample;
(d) detecting off-target activity of base editors;
(e) assessing specificity and fidelity of a CRISPR nuclease; and/or
(f) analysing the ratio of staggered to blunt-ended DSBs induced by a genome-editing nuclease, preferably, a CRISPR nuclease.

In particular, the computer program product may comprise instructions which, when the program is executed by a computer, cause the computer to carry out steps as described above for analysis of the sequencing results. It may be the Breaktag NGSpipe2go pipeline, the BreakinspectoR program or, preferably, a combination thereof.

Throughout the invention, the term "about" is intended to be understood as "+/- 10%". If "about" relates to a range, it refers to both lower and upper limit of the range. "A" is intended to mean "one or more", if not explicitly mentioned otherwise.

All literature cited herein is herewith fully incorporated. The present invention is further illustrated, but not limited, by the following examples.

### Figure legends

**Fig. 1****:** BreakTag workflow and adaptations. BreakTag starts with chromatin-free DNA in vitro digested with CRISPR nucleases or genomic DNA extracted from cells where CRISPR was delivered. Cleavage sites are end-repaired and labelled with custom linkers. Samples are tagmented with single-handle Tn5, and PCR is used to selectively enrich for cleavage events.
**Fig. 2****:** Exemplary designs of **(A)** the first linker (BreakTag linker, SEQ ID NO: 10 and 11) and **(B)** the second linker (SEQ ID NO: 7) used for the method according to the invention. The design of the first linker as shown in (A) is based on the commercially available Read 1-Nextera Transposase Adapter. The ME (mosaic end) region and the Tn5 A region form a first partial sequencing adapter region comprising a first sequencing primer binding site. The Tn5 A region denotes a nucleic acid sequence, which serves as a first PCR handle for the attachment of a first full-length sequencing adapter. The second linker corresponds to the commercially available Read 2-Nextera Transposase Adapter. The ME region and the Tn5 B region form a second partial sequencing adapter comprising a second sequencing primer binding site. The Tn5 B region denotes a nucleic acid sequence, which serves as a second PCR handle for the attachment of a second full-length sequencing adapter.

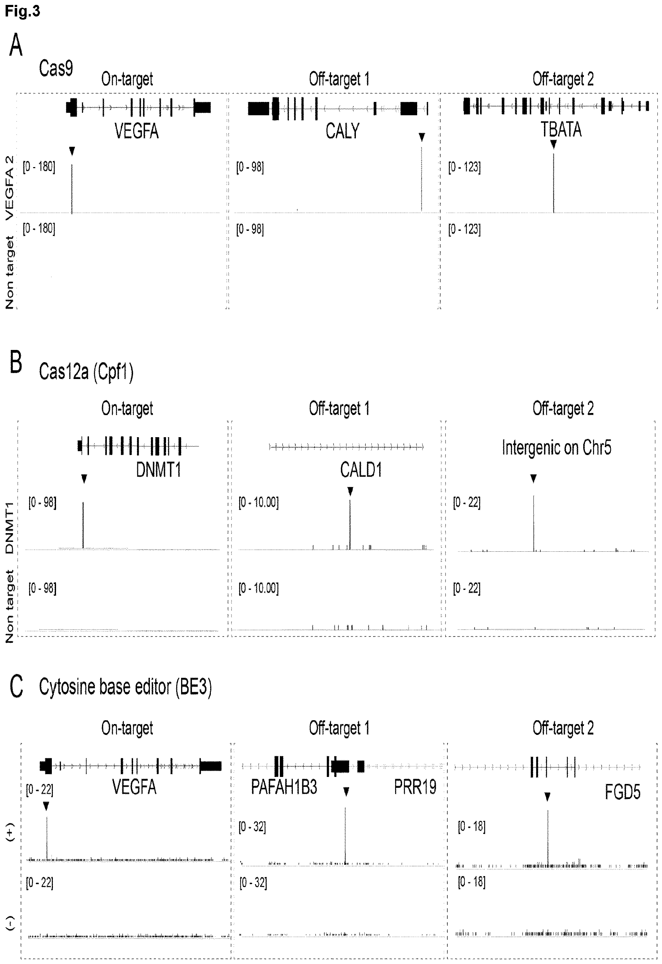

**Fig. 3****:** After sequencing and data analysis, genomic tracks can be generated for visualization in a genome browser. BreakTag can be applied to any CRISPR nuclease, as well as base-editors by adding a simple step in the workflow in order to convert sites of base editing.

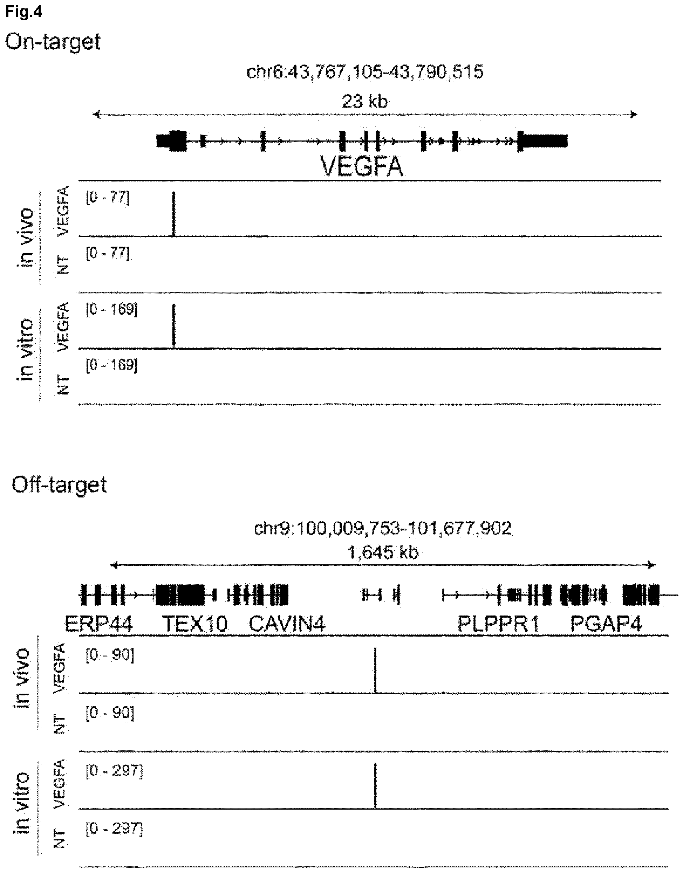

**Fig. 4****:** BreakTag profiles DSBs induced by Cas9 both in vitro and in vivo. A gRNA targeting the VEGFA site 2 was used in experiments for in vitro Cas9 digestion or was electroporated in vivo in HEK293T cells expressing Cas9.

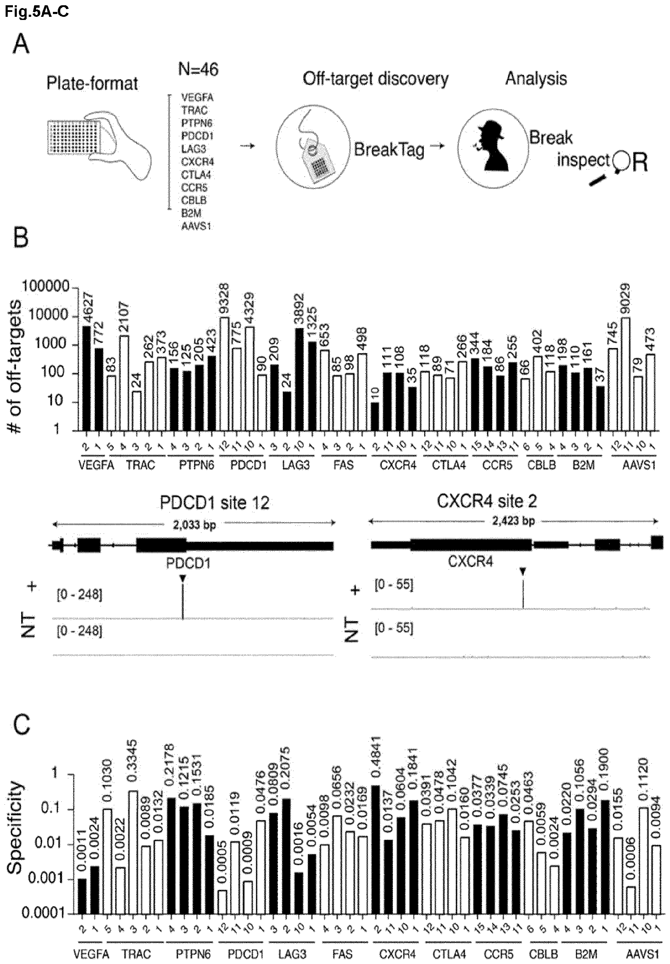

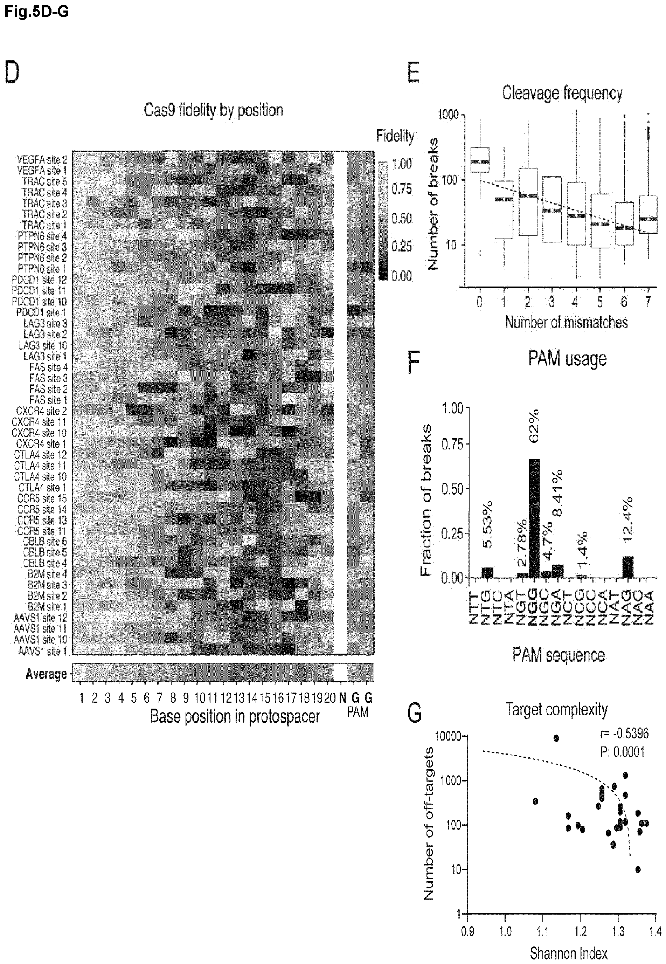

**Fig. 5****.** High-throughput investigation of sgRNA specificity and mismatch tolerance. (a) BreakTag was performed in a plate-format using a panel of 46 sgRNAs targeting clinically relevant genes and BreakinspectoR was used for off-target identification. (b) Number of off-targets was calculated using BreakinspectoR. (c) Bar plot showing specificity ratio (log scale). (d) Position of protospacer mismatches at off target sequences relative to homologous genomic sites. (e) Bar plot showing number of DSBs measured by BreakTag relative to the number of mismatches identified at off-targets relative to target site. (f) Bar plot of average BreakTag read count fraction at off-target sites categorized by PAM sequence (n = 46). (g) Scatterplot showing correlation of nucleotide variability with number of BreakTag-detected off-target sites (log scale).

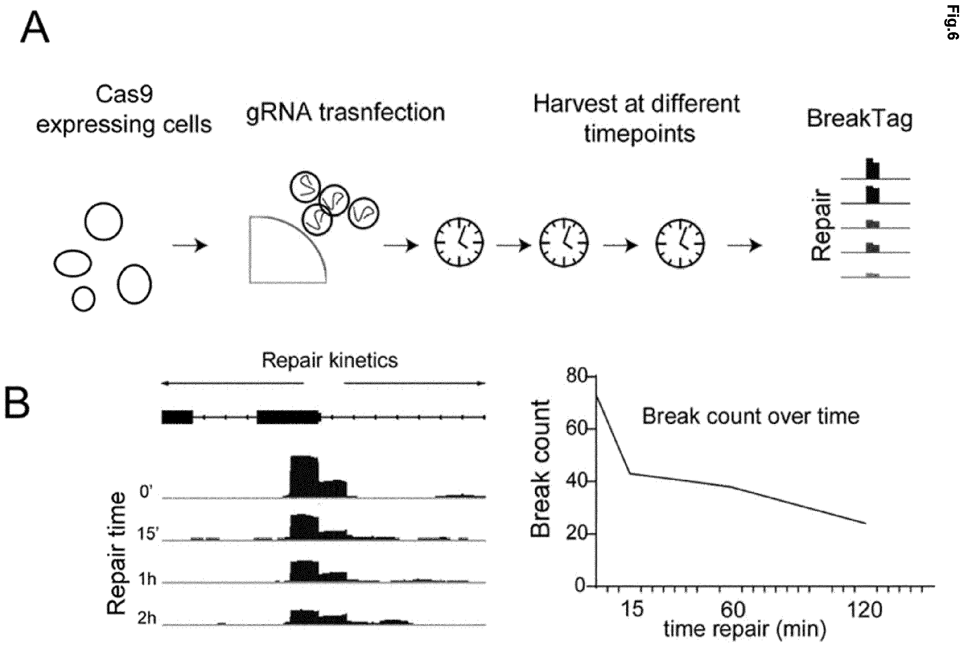

**Fig. 6****.** Repair kinetics of CRISPR breaks. (a) Experimental design of repair kinetics experiment in HEK-spyCas9 cells. Single guide RNAs were nucleofected in SpyCas9-expressing cells and gDNA was harvested at different timepoints. (b) IGV snapshot of on-target region of VEGFA site 2 showing DSB signal decrease overtime.

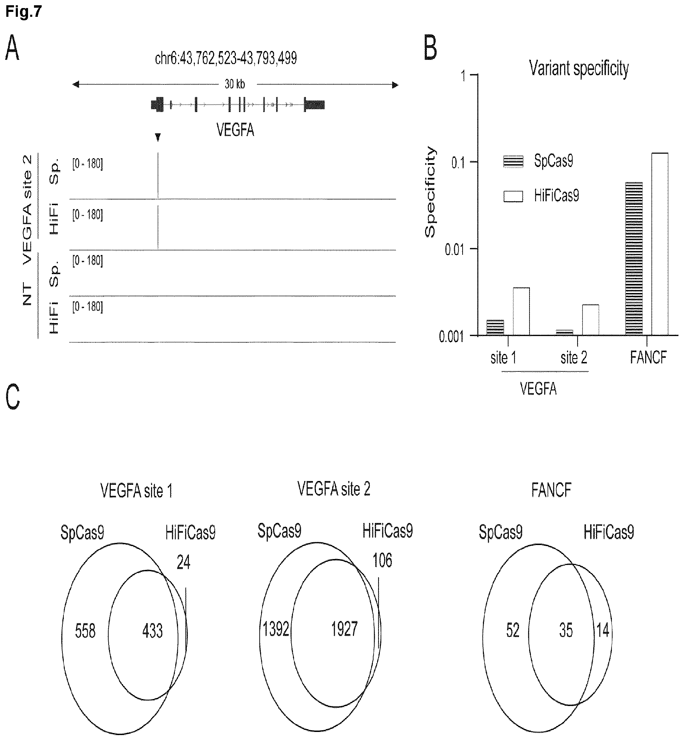

**Fig. 7****.** Specificity of engineered high fidelity Cas9 nuclease (HiFi) compared to SpCas9 evaluated by BreakTag. (a) On target DSBs (IGV snapshot) assessed by BreakTag in vitro induced by SpCas9 (Spy) or HiFi Cas9 targeting the VEGFA gene. (b) Specificity ratio of SpCas9 and HiFi Cas9 for three different gRNAs. (c) Venn diagrams (upper panel) and heatmaps of the number of DSBs across the genome (lower panels) identified by BreakTag for SpyCas9 and HiFi version using gRNAs for two sites in VEGFA gene and one in FANCF.

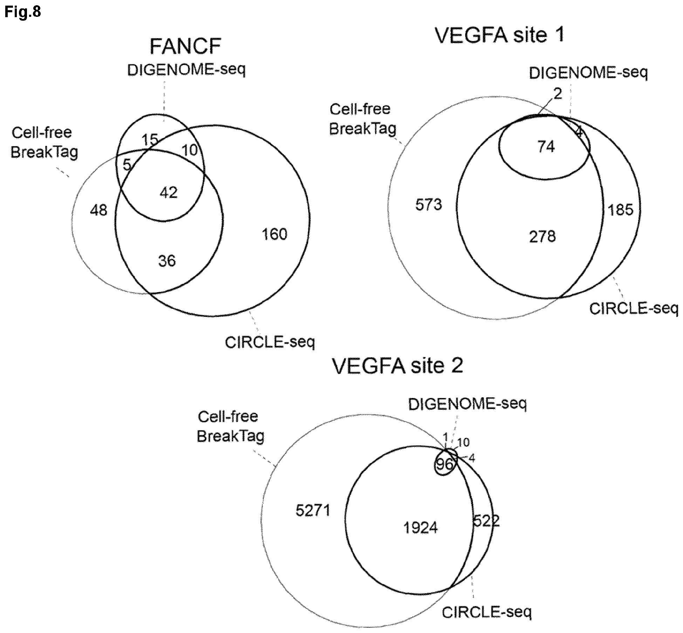

**Fig. 8****.** Overlapping and unique sites nominated by Multiplex DIGENOME-seq and CIRCLE-seq allowing sites with up to 6 mismatches.

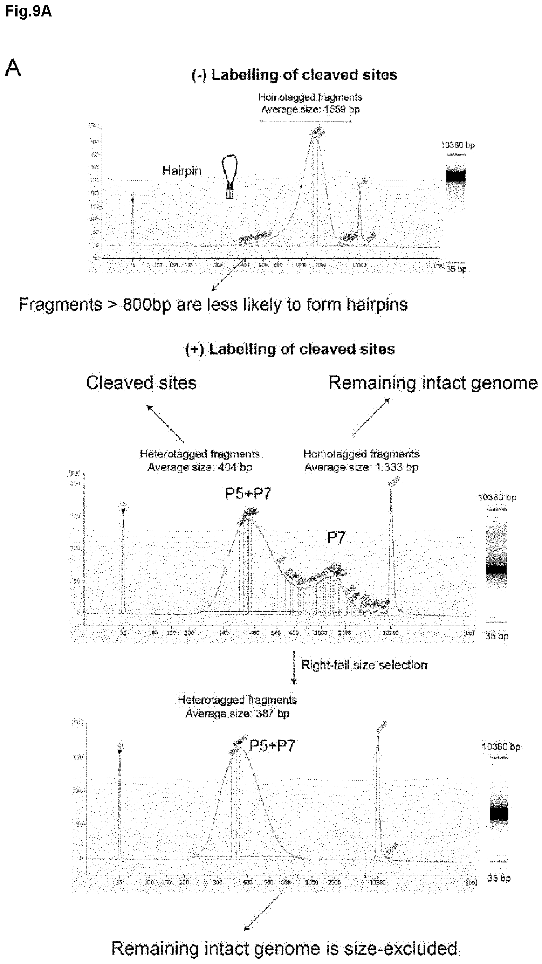

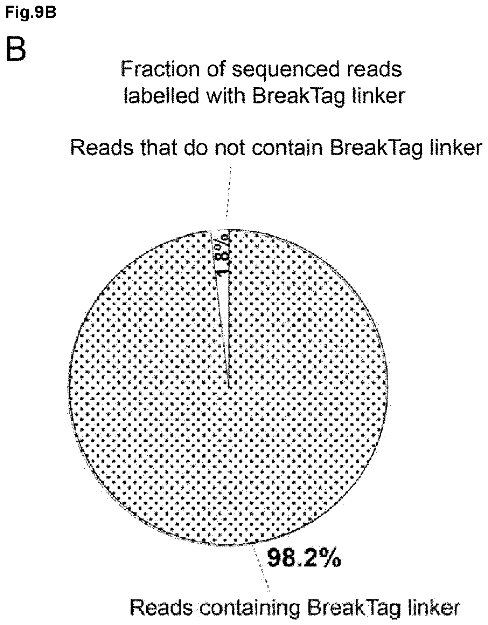

**Fig. 9****:** Enrichment of cleaved sites is achieved by a single-handle tagmentation followed by PCR. (a) Histograms show bioanalyzer traces of genomic DNA during BreakTag sample processing. (b) Reads containing BreakTag linker after demultiplexing. PhiX reads and reads that could not be assigned to an i5 or i7 index were excluded from the analysis.

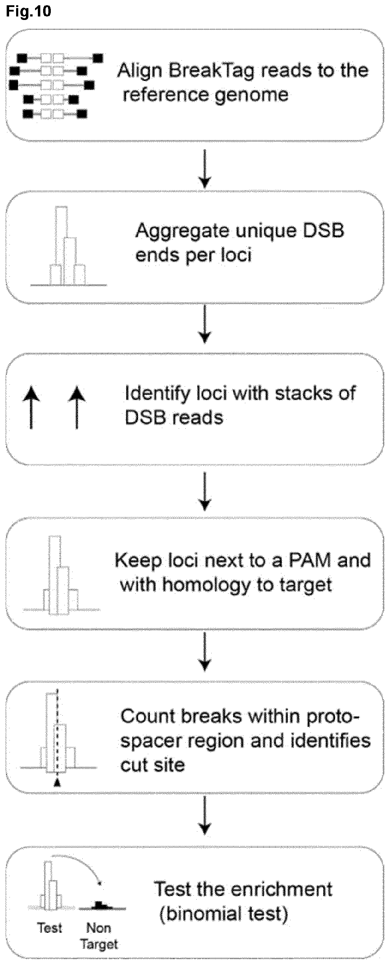

**Fig. 10****:** Schematic of the two-step analysis workflow for profiling on- and off-targets of a CRISPR nuclease using the Breaktag NGSpipe2go and BreakinspectoR pipelines.

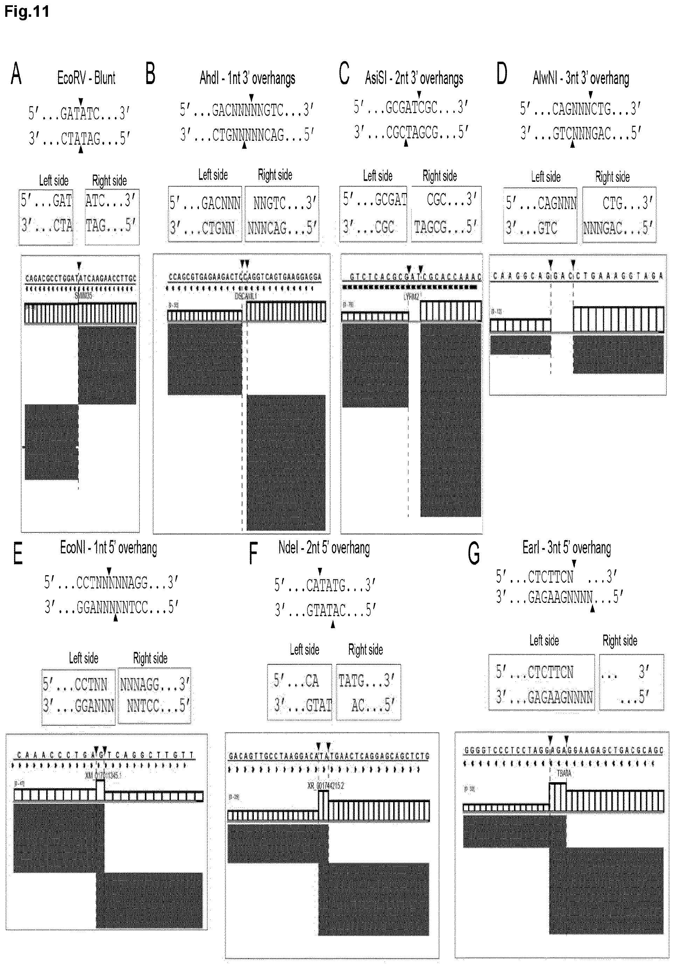

**Fig. 11****:** BreakTag reads signature reveals ssDNA DSB end. (A-G) Genomic DNA (SEQ ID NO: 12-18) of HEK293 was digested *in vitro* with different restriction enzymes for 1 hour at 37°C in NEB3.1 buffer, followed by BreakTag sample preparation. IGV snapshots from mapped reads (BAM) depicting gaps and overlaps between opposite reads of the DSB. These signatures are used to infer DSB end structure.

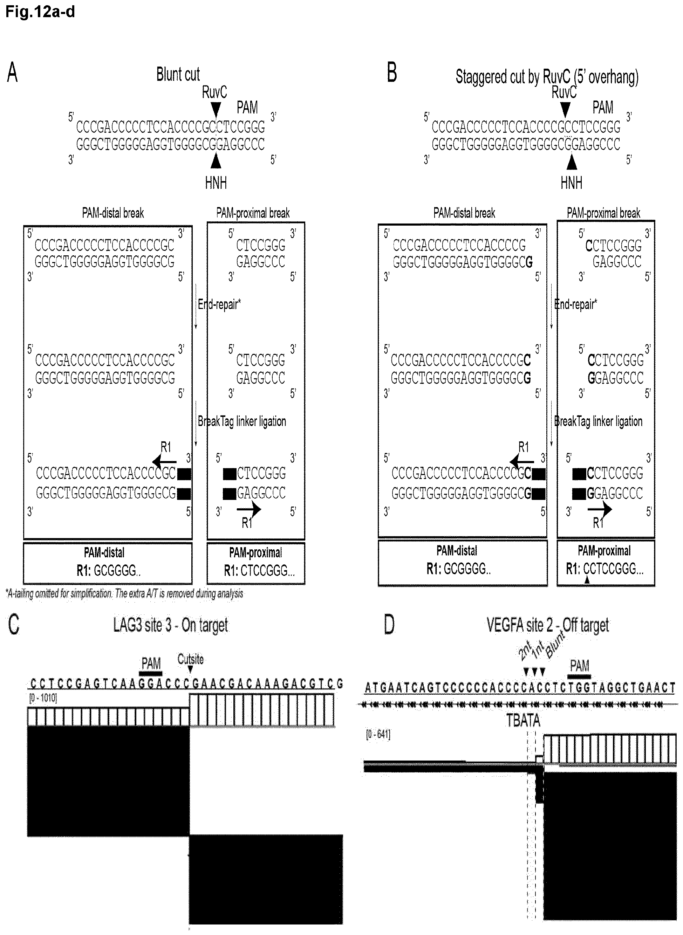

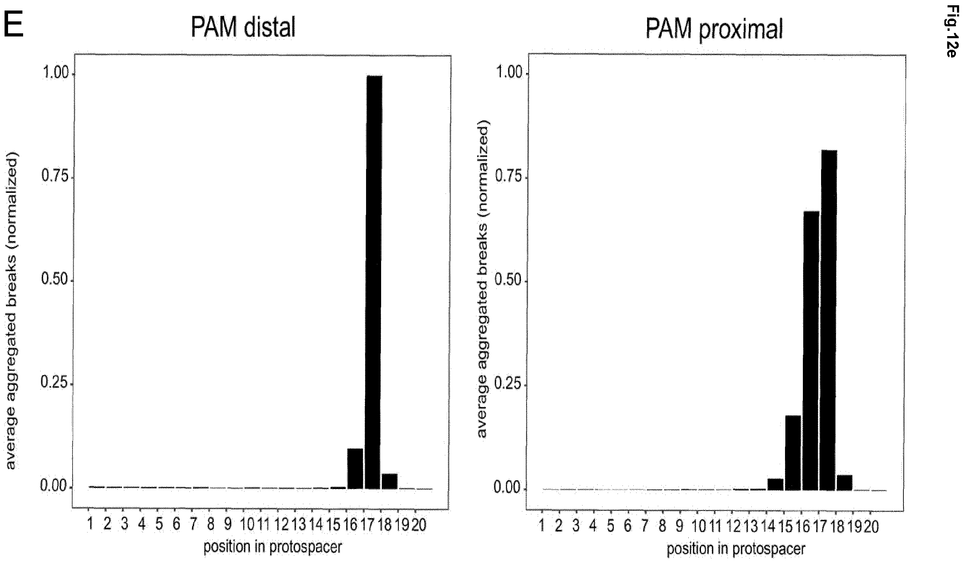

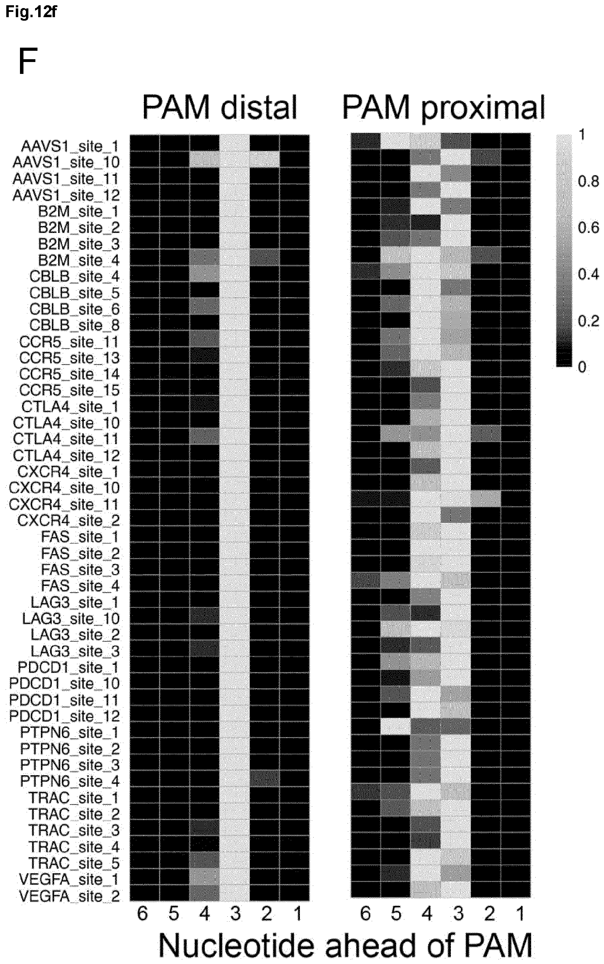

**Fig. 12****:** BreakTag read signatures reveal Cas9 scissile profile. (A) PAM-proximal and PAM-distal reads (SEQ ID NO: 19-22) of blunt cuts start at the same position. (B) 5' ssDNA overhangs are filled-in during BreakTag, PAM-proximal reads start at the last nucleotide of the ssDNA portion, whereas PAM-distal starts at the 4^{th} nucleotide (SEQ ID NO: 23-27). (C) IGV snapshot showing an example of a blunt cut (SEQ ID NO: 28). (D) IGV snapshot showing an example of an off-target with blunt, 1nt and 2nt 5' overhangs (SEQ ID NO: 29). (E) Barplots show positions of read accumulation along the protospacer for PAM-distal and PAM-proximal reads. (F) Heatmaps show positions of read accumulation for PAM-distal and PAM-proximal reads. Each row represents a different sgRNA used for cell-free BreakTag.

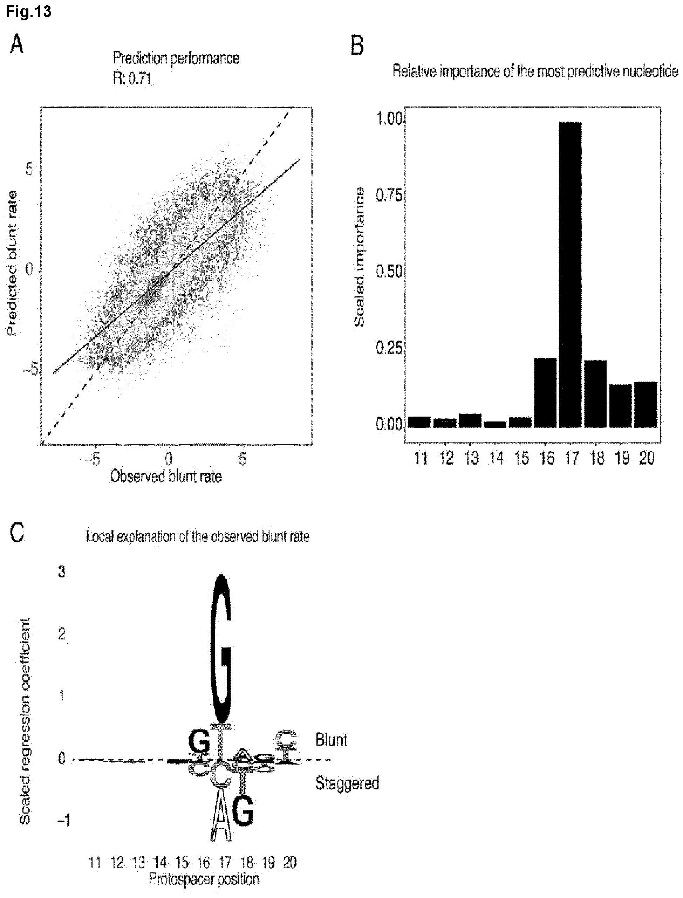

**Fig. 13****:** A machine learning approach identifies determinants of scissile profile. (A) Correlation between observed and predicted blunt rate. Dotted line shows perfect correlation. Continuous line shows correlation observed. (B) Relative importance of each position along the seed sequence of the protospacer. (C) Motif analysis showing representation of each base in either blunt or staggered breaks.

### Examples

### Example 1

### Materials

**The following materials were used for the experiments described below:**
Qiagen DNeasy Blood & Tissue Kit - Qiagen 69504
Thermolabile Proteinase K - NEB P8111S
RNase A, DNase and protease-free (10 mg/mL) - Thermo Scientific EN0531
Buffer NEB 3.1 - NEB B6003S
Alt-R^{®} S.p. Cas9 Nuclease V3 - IDT 1081058
Homemade SPRI magnetic beads or AMPure XP Reagent - Beckman Coulter A63880
NEBNext^{®} Ultra^{™} II End Repair/dA-Tailing Module - NEB E7546S
NEBNext^{®} Ultra^{™} II Ligation Module - NEB E7595S
Homemade hyperactive Tn5 or EZ-Tn5 Transposase - Lucigen TNP92110
NEBNext^{®} Ultra^{™} II Q5^{®} Master Mix - NEB M0544S
Qubit^{™} dsDNA High Sensitivity - Invitrogen Q32851
Agilent High Sensitivity DNA Kit - Agilent 5067-4626
DNA LoBind^{®} Tubes - Eppendorf 0030122348

### Methods

### 1. DNA extraction

Genomic DNA (gDNA) was extracted from cells using the DNeasy Blood & Tissue (Qiagen) following manufacturer's instructions.

### 2. gDNA digestion with Ribonucleoprotein (RNP) for Cell-free BreakTag

Nuclease was diluted to 1µM in NEB 3.1 buffer as follows:

| **Reagent** | **Volume (µL)** |
|---|---|
| NEB 3.1 10x | 6.2 |
| Nuclease (62µM) | 1 |
| H2O | 54.8 |
| FV | 62 |

Next, the sgRNA was diluted to 3µM in nuclease-free H₂O. The dilution was subsequently incubated at 95°C for 2 min, then left it at room temperature for 5 min.

The following reagents were mixed for RNP assembly:

| **Reagent** | **Vol 1 reaction (µL)** |
|---|---|
| NEB 3.1 10x | 5 |
| Cas9 nuclease (1µM dilution) | 4.5 |
| sqRNA (3µM dilution) | 1.5 |
| Total | 11 |

The mixture was subsequently incubated at 37°C for 10 min in a thermocycler.

11 µL of the assembled RNP mix were then added into 39µL of 12.85 ng/µL of DNA. The mixture was incubated for 1h at 37°C, then kept at 4°C;

4µL of RNAse A 10mg/mL (final 0.8µg/µL) and 1µL thermolabile of proteinase K 20mg/mL (final 0.4µg/µL) were added to the 50µL solution containing DNA + RNP. The mixture was incubated at 37°C for 20 min, followed by inactivation at 55°C for 20 min (in a thermocycler). Next a 1.4x AMpure bead clean-up was performed following manufacturer's instructions for incubation time and elution with ethanol. The DNA was finally eluted in 26µL of H₂O. DNA can be frozen at this point.

### 3. Oligos preparation

### Tagmentation linker and transposome assembly:

Tagmentation linker was prepared by mixing 100 µM of Tn5ME-B (Illumina FC-121-1031) and 100 µM Tn5MErev resuspended in annealing buffer (50mM NaCl, 40mM Tris pH 8) at a 1:1 ratio. The oligos were annealed in a thermocycler as follows:

| Step | Temperature | Time |
|---|---|---|
| 1 | 95_{°}C | 5 minutes |
| 2 | 65_{°}C | -0.1_{°}C/second |
| 3 | 65_{°}C | 5 minutes |
| 4 | 4_{°}C | -0.1_{°}C/second |
| 5 | 4_{°}C | Hold |

Homemade Tn5 was loaded with pre-annealed Tn5 B adapter for 1 hour at room temperature with 300 RPM agitation in a thermoshaker.

### BreakTag linker preparation:

BreakTag linker was prepared by combining 10 µM of BreakTag_1_fwd and 10 µM of BreakTag_1_rev oligos in T4 Polynucleotide Kinase buffer (NEB M0201S). The oligos were annealed in a thermocycler with the following program:

| Step | Temperature | Time |
|---|---|---|
| 1 | 95_{°}C | 5 minutes |
| 2 | Cool to 25_{°}C | -0.1_{°}C/second |
| 3 | 25_{°}C | Hold |

### 4. End-prep:

25µL of DNA were dispensed in nuclease-free H₂O (500ng total input) in PCR strips, tubes or PCR plates. Next, the following mix was prepared in a 1.5mL eppendorf tube:

| **Component** | **1 reaction (µL)** |
|---|---|
| End prep reaction buffer | 3.5 |
| End prep Enzyme | 1.5 |

Then, 5µL of the mix were added per tube containing 25µL of DNA in H₂O. The reaction was subsequently placed in a thermocycler, with the heated lid set to ≥ 75°C, and the following program was run:
30 minutes at 20°C
30 minutes at 65°C
Hold at 4°C

### 5. BreakTag linker ligation:

The previously annealed BreakTag linker was diluted in nuclease-free H₂O for a final concentration of 5µM (stock is at 10µM). 1µL of linker was then added at 5µM in each sample. Afterwards, the following mix was prepared on ice (please note that it is not recommended to prepare the ligation mix containing the BreakTag linker because it increases linker concatamerization):

| **Component** | **1 reaction (µL)** |
|---|---|
| H₂O | 0.25 |
| Ultra II ligation mix | 15 |
| Liq. enhancer | 0.5 |

Then, 15.75 µL of the mix were added in each tube containing 30µL of end-prep DNA. Next, the following program was run in a thermocycler with the lid heating off:
15 minutes @ 20°C
15 minutes @37°C

Then, 2 consecutive rounds of a 0.7x volumes left-tail size selection were performed using AMpure XP beads in order remove unligated fragments while following the manufacturer's instructions for ethanol washes and incubation times. The DNA was subsequently eluted in 12µL of nuclease-free H₂O and 1µL of sample was used for Qubit quantification.

### 6. Tagmentation:

An in-house production of hyperactive Tn5 was used (see Hennig et al., 2018). The tagmentation buffer 4x contained: 40mM Tris-CI pH 7.5, 40mM MgCl2 in H2O and was stable for a few weeks at RT.

| **Component** | **1 reaction (µL)** |
|---|---|
| Fresh taqmentation buffer 2x (50% DMF) | 10 |
| Labeled DNA | 5 |
| Tn5 dilution in H₂O* | 5 |

| | |
|---|---|
| **The ratio of Tn5 to DNA has to be tested empirically* | |

The samples were incubated in a pre-heated thermocycler at 55°C for 5 minutes, then kept at 12°C before proceeding immediately to the reaction termination. To stop the reaction, 5µL of SDS 0.2% (freshly diluted) was added and vortexed well before incubation for 5 min at RT. Immediately afterwards, PCR was performed.

### 7. PCR:

The PCR reaction was assembled by mixing the following components:

| **Component** | **1 reaction (µL)** |
|---|---|
| Taqmented DNA | 25 |
| I5 index primer (10µM) | 5 |
| I7 index primer (10µM) | 5 |
| Ultra II Q5 MM 2x | 35 |

The PCR reactions were split into 2 tubes containing 35µL each and the following PCR program was run:

| **Step** | **Temperature** | **Time** | |
|---|---|---|---|
| 1 | 72_{°}C | 5 minutes | Gap filling reaction |
| 2 | 98_{°}C | 30 seconds | |
| 3 | 98_{°}C | 10 seconds | 14x |
| 4 | 63_{°}C | 30 seconds | |
| 5 | 72_{°}C | 60 seconds | |
| 6 | 72_{°}C | 5 minutes | |
| 7 | 12_{°}C | Hold | |

### 8. Library clean-up:

2 rounds of the following size-selection scheme were performed using AMPure XP beads: 0.5x right-tail clean-up (removes fragments >1kb) + 0.85X left-tail (removes fragments <200bp). The manufacturer's instructions were followed for ethanol washes and incubation times.

### 9. DNA sequencing

For sequencing, libraries were pooled at equimolar concentrations and run in a NextSeq550 high output flow cell single-read 75bp, dual indexing. For this, the manufacturer's instructions were followed. If the same sample barcode sequence was used for all samples, 15% PhiX were spiked-in to pool prior to loading to ensure nucleotide diversity on cycles 9-16.

### Oligonucleotide sequences:

| **Name** | **Sequence 5'-3'** |
|---|---|
| Tn5MErev | /5Phos/CTGTCTCTTATACACATCT (SEQ ID NO: 5) |
| Tn5ME-B | GTCTCGTGGGCTCGGAGATGTGTATAAGAGACAG (SEQ ID NO: 7) |
| BTLinker_1_rev | CGATTGAGGCCGGGTCTCGTCGGCAGCGTCAGATGTG- TATAAGAGACAGNNNNNNNNCTCACACGT (SEQ ID NO: 3) |
| BTLinker_1_fwd | /5phos/CGTGTGAGNNNNNNNNCTGTCTCT- TATACACATCTGACGCTGCCGACGAGACCCGGCCTCA- ATCGAA (SEQ ID NO: 4) |
| N7XX (Primer adding the p7 sequencinq adapter) | CAAGCAGAAGACGGCATACGAGATNNNNNNNN- GTCTCGTGGGCTCGG (SEQ ID NO: 9) |
| S5XX (Primer adding the p5 sequencing adapter) | AATGATACGGCGACCACCGAGATCTACACNNNNNNNN- NTCGTCGGCAGCGTC (SEQ ID NO: 8) |
| | |
| /5phos/ indicates 5' phosphorylation | |
| Underscored sequence indicates sample barcode in BTLinker fwd and rev oligonucleotides | |
| NNNNNNNN indicates UMI in BTLinker fwd and rev oligonucleotides and i5/i7 index sequences in N7XX and S5XX indexed primers | |

### sgRNA sequences:

| **Sequence Name** | **Sequence** |
|---|---|
| AAVS1_site_1 (SEQ ID NO: 30) | mG*mU*mC* rArCrC rArArU rCrCrU rGrUrC rCrCrU rArGrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| CBLB_site_4 (SEQ ID NO: 31) | mG*mG*mC* rArGrA rArArC rCrCrU rGrGrU rGrGrU rCrGrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| CTLA4_site_1 | mG*mC*mA* rCrGrU rGrGrC rCrCrA rGrCrC rUrGrC rUrGrG rUrUrU rUrArG rArGrC rUrArG rArArA rUrArG rCrArA rGrUrU rArArA rArUrA |
| (SEQ ID NO: 32) | rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| FAS_site_1 (SEQ ID NO: 33) | mG*mA*mG* rGrGrC rUrCrA rCrCrA rGrArG rGrUrA rGrGrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| PDCD1_site_1 (SEQ ID NO: 34) | mG*mG*mC* rGrCrC rCrUrG rGrCrC rArGrU rCrGrU rCrUrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| TRAC_site_1 (SEQ ID NO: 35) | mG*mU*mC* rArGrG rGrUrU rCrUrG rGrArU rArUrC rUrGrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| AAVS1_site_10 (SEQ ID NO: 36) | mG*mG*mG* rArArC rCrCrA rGrCrG rArGrU rGrArA rGrArG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| CBLB_site_5 (SEQ ID NO: 37) | mG*mA*mU* rUrUrC rCrUrC rCrUrC rGrArC rCrArC rCrArG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| CTLA4_site_10 (SEQ ID NO: 38) | mG*mA*mG* rGrUrU rCrArC rUrUrG rArUrU rUrCrC rArCrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| FAS_site_2 (SEQ ID NO: 39) | mG*mC*mU* rGrArC rCrCrC rGrCrU rGrGrG rCrArG rGrCrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| PDCD1_site_10 (SEQ ID NO: 40) | mG*mG*mA* rGrArA rGrGrU rGrGrG rGrGrG rGrUrU rCrCrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| TRAC_site_2 (SEQ ID NO: 41) | mG*mC*mU* rGrGrU rArCrA rCrGrG rCrArG rGrGrU rCrArG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| AAVS1_site_11 (SEQ ID NO: 42) | mG*mG*mU* rGrArG rGrGrA rGrGrA rGrArG rArUrG rCrCrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| CBLB_site_6 (SEQ ID NO: 43) | mG*mG*mA* rUrUrU rCrCrU rCrCrU rCrGrA rCrCrA rCrCrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| CTLA4_site_11 (SEQ ID NO: 44) | mG*mG*mC* rCrArG rUrArC rCrArC rArGrC rArGrG rCrUrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| FAS_site_3 (SEQ ID NO: 45) | mG*mG*mG* rGrCrA rGrCrU rCrCrG rGrCrG rCrUrC rCrUrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| PDCD1_site_11 (SEQ ID NO: 46) | mG*mA*mA* rGrGrU rGrGrC rGrUrU rGrUrC rCrCrC rUrUrG rUrUrU rU- rArG rArGrC rUrArG rArArA rUrArG rCrArA rGrUrU rArArA rArUrA rArGrG |
| | rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| TRAC_site_3 (SEQ ID NO: 47) | mG*mA*mG* rArArU rCrArA rArArU rCrGrG rUrGrA rArUrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| AAVS1_site_12 (SEQ ID NO: 48) | mG*mC*mG* rCrCrG rArGrA rArGrG rArArG rUrGrC rUrCrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| CBLB_site_8 (SEQ ID NO: 49) | mG*mG*mG* rUrArU rUrArU rUrGrA rUrGrC rUrArU rUrCrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| CTLA4_site_12 (SEQ ID NO: 50) | mG*mC*mA* rCrArA rGrGrC rUrCrA rGrCrU rGrArA rCrCrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| FAS_site_4 (SEQ ID NO: 51) | mG*mU*mG* rGrArG rCrGrC rArGrU rGrGrU rCrUrC rCrGrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| PDCD1_site_12 (SEQ ID NO: 52) | mG*mA*mG* rArArG rGrUrG rGrGrG rGrGrG rUrUrC rCrArG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| TRAC_site_4 (SEQ ID NO: 53) | mG*mA*mC* rArCrC rUrUrC rUrUrC rCrCrC rArGrC rCrCrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| B2M_site_1 (SEQ ID NO: 54) | mG*mG*mC* rCrGrA rGrArU rGrUrC rUrCrG rCrUrC rCrGrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| CCR5_site_11 (SEQ ID NO: 55) | mG*mG*mC* rArGrC rArUrA rGrUrG rArGrC rCrCrA rGrArG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| CXCR4_site_1 (SEQ ID NO: 56) | mG*mA*mU* rArArC rUrArC rArCrC rGrArG rGrArA rArUrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| LAG3_site_1 (SEQ ID NO: 57) | mG*mA*mU* rGrUrG rGrGrA rGrGrC rUrCrA rGrUrU rCrCrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| PTPN6_site_1 (SEQ ID NO: 58) | mG*mG*mU* rGrGrA rUrGrA rUrGrG rUrGrC rCrGrU rCrGrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| TRAC_site_5 (SEQ ID NO: 59) | mG*mU*mC* rGrArG rArArA rArGrC rUrUrU rGrArA rArCrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| B2M_site_2 (SEQ ID NO: 60) | mG*mC*mU* rArCrU rCrUrC rUrCrU rUrUrC rUrGrG rCrCrG rUrUrU rU- rArG rArGrC rUrArG rArArA rUrArG rCrArA rGrUrU rArArA rArUrA rArGrG |
| | rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| CCR5_site_13 (SEQ ID NO: 61) | mG*mA*mC* rArUrU rArArA rGrArU rArGrU rCrArU rCrUrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| CXCR4_site_10 (SEQ ID NO: 62) | mG*mC*mC* rGrUrG rGrCrA rArArC rUrGrG rUrArC rUrUrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| LAG3_site_10 (SEQ ID NO: 63) | mG*mG*mG* rCrUrG rCrArG rGrGrG rArGrC rUrGrG rGrCrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| PTPN6_site_2 (SEQ ID NO: 64) | mG*mG*mG* rArUrC rArGrG rUrGrA rCrCrC rArUrA rUrUrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| VEGFA site-seq (SEQ ID NO: 65) | mG*mG*mG* rUrGrG rGrGrG rGrArG rUrUrU rGrCrU rCrCrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| B2M_site_3 (SEQ ID NO: 66) | mG*mA*mG* rUrArG rCrGrC rGrArG rCrArC rArGrC rUrArG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| CCR5_site_14 (SEQ ID NO: 67) | mG*mG*mU* rGrArC rArArG rUrGrU rGrArU rCrArC rUrUrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| CXCR4_site_11 (SEQ ID NO: 68) | mG*mU*mA* rCrArG rGrCrU rGrCrA rCrCrU rGrUrC rArGrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| LAG3_site_2 (SEQ ID NO: 69) | mG*mC*mU* rGrUrU rUrCrU rGrCrA rGrCrC rGrCrU rUrUrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| PTPN6_site_3 (SEQ ID NO: 70) | mG*mA*mU* rUrUrC rUrArU rGrArC rCrUrG rUrArU rGrGrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| VEGFA site2 (SEQ ID NO: 71) | mG*mA*mC* rCrCrC rCrUrC rCrArC rCrCrC rGrCrC rUrCrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| B2M_site_4 (SEQ ID NO: 72) | mG*mG*mC* rCrArC rGrGrA rGrCrG rArGrA rCrArU rCrUrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| CCR5_site_15 (SEQ ID NO: 73) | mG*mC*mU* rGrUrG rUrUrU rGrCrG rUrCrU rCrUrC rCrCrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| CXCR4_site_2 (SEQ ID NO: 74) | mG*mC*mA* rUrUrU rUrCrU rUrCrA rCrGrG rArArA rCrArG rUrUrU rUrArG rArGrC rUrArG rArArA rUrArG rCrArA rGrUrU rArArA rArUrA rArGrG |
| | rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| LAG3_site_3 (SEQ ID NO: 75) | mG*mC*mU* rGrCrA rGrArA rArCrA rGrCrA rArGrC rCrCrG rUrUrU rUrArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| PTPN6_site_4 (SEQ ID NO: 76) | mG*mG*mU* rUrUrC rArCrC rGrArG rArCrC rUrCrA rGrUrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| Non-targeting (BFP) (SEQ ID NO: 77) | mG*mC*mU* rGrArA rGrCrA rCrUrG rCrArC rGrCrC rArUrG rUrUrU rU- rArG rArGrC rUrArG rArArA rUrArG rCrArA rGrUrU rArArA rArUrA rArGrG rCrUrA rGrUrC rCrGrU rUrArU rCrArA rCrUrU rGrArA rArArA rGrUrG rGrCrA rCrCrG rArGrU rCrGrG rUrGrC mU*mU*mU* rU |
| | "*" indicates phosphorothioate modified base |

### Results:

### 1. High throughput assessment of the off-target landscape across 46 guide RNAs

We performed **BreakTag** in a panel of 46 different gRNAs sequences across 11 clinically-relevant genes for cancer treatment and CAR-T cell therapy **(****Figure 5a****)** *in vitro.* Guide sequences showed a large of cleaved sites, ranging from 10 for the CXCR4 site 2 to 9.328 for PDCD1 site 12 **(****Figure 5b****).** Our software **BreakinspectoR** reports numerous outputs that allow the user to evaluate the specificity of their experimental gRNAs. Genome-wide analyses of the frequency and location of induced DSBs, identified in addition to the predicted on target sites a total of 45.563 off-targets. **BreakTag** specificity *ratio,* define as the sum of on-target reads by the sum of off-targets, was able to identify target sites with high specificity **(****Figure 5c****).** In order to evaluate factors influencing Cas9 activity at individual off-target sites, we analyzed the effects of number of mismatches, their position within the protospacer and nature of the base, producing individual Cas9 fidelity maps. Interestingly, while each gRNA showed an individual pattern, at average, nucleotide mismatches at off-targets were more frequently at the PAM-distal region of the target sequence, whereas the PAM-proximal region was less permissive to incorrect base-pairing **(****Figure 5d****).** As expected, there was an inverse correlation between the target cleavage frequency and the number of mismatches at the protospacer sequence **(****Figure 5e****).** At off-target sites genome-wide, canonical NGG PAMs were cleaved with the highest average frequency, followed by NAG, NGA and NTG **(****Figure 5f****),** and higher nucleotide diversity within the target sequence showed propensity for lower off-target numbers **(****Figure 5g****).**

Our comprehensive characterization of the off-target landscape allows the user to modify their experimental guide RNA sequences in order to reduce off-target effect. For example, it has been previously shown that addition of 2-deoxynucleotides at the gRNA sequence reduce off-target activity by reducing mismatch tolerance (Donohoue et al., 2021), and we speculate that chemical modifications at the site of mismatch accumulation will have a greater mitigatory effect in off-target cleavage.

### 2. Using BreakTag to follow repair kinetics of CRISPR breaks in living cells.

We used **BreakTag** to investigate the repair kinetics of CRISPR breaks. We delivered sgRNA sequences to Cas9-expressing HEK293T cells, and gDNA was extracted at different timepoints for **BreakTag (****Figure 6a****).** We observe a gradual reduction of Cas9 breaks over time at the on-target **(****Figure 6b****)** as well as the off-targets. This data shows that **BreakTag** could be used for the investigation of repair dynamics of Cas9 breaks in cells in a high-throughput manner.

### 3. Using BreakTag to assess of specificity of engineered high-fidelity Cas9 nucleases

Numerous engineered Cas9 nucleases have been generated that promise reduced number of CRISPR off-targets and thus higher specificity. The scalability, precision and extremely fast workflow of **BreakTag** are advantageous on assessing new variants in high-throughput manner. As proof-of-principle, we have used **BreakTag** to compare the specificity of an engineered, high fidelity version of Cas9 (HiFi Cas9) (Vakulskas et al., 2018) **(****Figure 7a****-c).** We found that the HiFi version showed higher specificity and reduced number of off-targets, while the efficiency of targeting the intended site was unaffected **(****Figure 7a****).**

### 4. Comparison with in vitro-based tools

*In vitro* methods for off-target discovery are highly sensitive, and provide a larger list of off-targets due to the lack of chromatin context in the assay. Of note, not all off-targets nominated by the different methods are shared amongst each other, meaning that each method reports a unique set of off-targets presumably due to the different enrichment strategies (Atkins et al., 2021; Chaudhari et al., 2020). This discrepancy highlights the importance of interrogating the off-target landscape *in vitro* and at the cell-based level for the validation of bona-fide off-targets (Atkins et al., 2021). We compared the off-targets called by DIGENOME-seq (Kim et al., 2016) and CIRCLE-seq (Tsai et al., 2017) with BreakTag for the highly promiscuous VEGFA site 1 and VEGFA site 2. We observed that BreakTag captured the majority of off-targets found with SITE-seq and DIGENOME-seq **(****Figure 8****).**

### Example 2

There is an urgent need to elucidate sequence determinants of scissile profile, as this will allow better gRNA design for precise template-free Cas9 gene editing. Here, we used BreakTag to probe the frequency by which Cas9 coupled with different sgRNAs generate blunt and staggered cuts across the genome. First, using restriction enzymes with known cut patterns, we confirmed that BreakTag is able to probe and quantify the scissile profile of Cas nucleases and upgrade breakinpectoR to be able to identify and quantify Cas9 scissile pattern by focusing at the informative PAM proximal strand (see below). Then, in order to be able to identify sequence determinants that dictate the scissile profile of Cas9, we deployed artificial intelligence by training a machine learning model with a robust dataset of scissile-aware DSBs.

### BreakTag read signatures reveal DSB end structure.

Because BreakTag has single-nucleotide resolution and contains an end-repair step where 3' ssDNA overhangs are chewed back and 5' ssDNA overhangs are filled-in, we hypothesized that a shift in the position where DSB reads start would occur when ssDNA ends are formed. In order to validate this, we digested gDNA with restriction enzymes that generate blunt or 1-3nt long 5' or 3' overhangs and performed BreakTag (Fig. 11A-G).

As expected, symmetric reads derived from EcoRV cuts (blunt) started at the same position indicating no formation of ssDNA overhangs (Fig. 11A). When 3' ssDNA overhang restriction enzymes were used (Ahdl, AsiSI and AlwNI), clear gaps between DSB reads were observed due to resection of 3' overhangs (Fig. 11B-D). On the other hand, 5' overhang restriction enzymes (EcoNI, Ndel and Earl) generated overlapping reads, revealing that 5' overhangs were filled-in (Fig. 11E-F). Altogether, we conclude that BreakTag reveals the formation of ssDNA DSB end structures based on signature shift on read start locations.

### PAM-proximal reads reveal Cas9 flexible scissile profile.

Cas9-mediated DSBs contain two distinct sides, one containing the PAM-sequence (PAM-proximal) and the other containing the remaining protospacer sequence (PAM-distal) (Fig. 12a). When blunt cuts are formed, the PAM-proximal side of the break starts at the canonical 3^{rd} nt ahead of PAM whereas the PAM-distal read starts at 4^{th} nt (Fig. 12A,C), indicating that both strands were cleaved at the same position. Where 5' overhangs are formed, the PAM-distal read still maps at the 4^{th} nt, as the fill-in reaction occurs towards the PAM-sequence (Fig. 12B). However, the PAM-proximal read start position is shifted ahead, as the fill-in reaction occurs in the opposite direction of PAM (Fig. 12B,D).

In order to assess the frequency of blunt or staggered cuts by Cas9, we analyzed the signal accumulation for the PAM-proximal and PAM-distal reads for 42,657 on/off-targets generated from 47 sgRNAs with BreakTag. As expected, the great majority of PAM-distal reads started at the canonical 3^{rd} nucleotide ahead of PAM whereas PAM-proximal reads mostly accumulated at the 3^{rd} nucleotide, but also 4^{th}, 5^{th} and 6^{th}, indicating that 1-3nt 5' overhangs were generated by Cas9 as previously shown (Fig. 12F,G; Shi et al., 2019). Moreover, scissile profile was sequence-dependent, as different sgRNAs showed strikingly distinct cut signatures (Fig. 12G).

### Sequence determinants of scissile profile decision

In order to investigate sequence determinants of Cas9 scissile profile, we trained a gradient boosting regressor of 1,000 trees with XGBoost (eXtreme Gradient Boosting) to predict the scissile profile information of 21,413 cleaved sites identified with BreakTag over 47 sgRNAs for which BreakTag captured enough information to distinguish the scissile profile (cleaved sites with at least 8 reads mapping to the PAM proximal strand). As predictor variables, we used the one-hot-encoding of the pairwise alignment of the last 10 nucleotides of the protospacer sequence and the actual sgRNA used to guide Cas9, totaling 160 input parameters. The accuracy of the model was assessed with 5-folds of cross-validation.

The trained model showed a good correlation between observed and predicted blunt rates (R=0.71) (Fig. 13a). We observed that the 4 nucleotides near the PAM (20-16) showed the highest relative importance compared to further positions (Fig. 13b), suggesting that sequence composition around the cutsite is a major determinant of Cas9 scissile decision. We further investigated the sequence composition of blunt and staggered breaks at the most important positions. Interestingly, a strong bias towards G and T nucleotides at position 17 (3^{rd} NT ahead of PAM) was observed for staggered cuts (Fig. 13c). This important finding is a major step towards better design of gRNA sequences for precise Cas9 gene editing as repair of an overhang may result in a predictable templated insertion, making it more desirable for precise, predictable genome-editing.

### References:

Atkins A, Chung C-H, Allen AG, et al. Off-Target Analysis in Gene Editing and Applications for Clinical Translation of CRISPR/Cas9 in HIV-1 Therapy. Front Genome Ed. 2021;3(August): 1-26. doi:10.3389/fgeed.2021.673022
Cameron P, Fuller CK, Donohoue PD, et al. Mapping the genomic landscape of CRISPR-Cas9 cleavage. Nat Methods. 2017;14(6):600-606. doi:10.1038/nmeth.4284
Tsai SQ, Nguyen NT, Malagon-Lopez J, Topkar V V., Aryee MJ, Joung JK. CIRCLE-seq: A highly sensitive in vitro screen for genome-wide CRISPR-Cas9 nuclease off-targets. Nat Methods. 2017;14(6):607-614. doi:10.1038/nmeth.4278
Lazzarotto CR, Malinin NL, Li Y, et al. CHANGE-seq reveals genetic and epigenetic effects on CRISPR-Cas9 genome-wide activity. Nat Biotechnol. 2020;38(11):1317-1327. doi:10.1038/s41587-020-0555-7
Tsai SQ, Zheng Z, Nguyen NT, et al. GUIDE-seq enables genome-wide profiling of off-target cleavage by CRISPR-Cas nucleases. Nat Biotechnol. 2015;33(2):187-198. doi:10.1038/nbt.3117
Schmid-Burgk JL, Gao L, Li D, et al. Highly Parallel Profiling of Cas9 Variant Specificity. Mol Cell. 2020;78(4):794-800.e8. doi:10.1016/j.molcel.2020.02.023
Wienert B, Wyman SK, Yeh CD, Conklin BR, Corn JE. CRISPR off-target detection with DISCOVER-seq. Nat Protoc. 2020;15(5):1775-1799. doi:10.1038/s41596-020-0309-5
Canela A, Sridharan S, Sciascia N, et al. DNA Breaks and End Resection Measured Genome-wide by End Sequencing. Mol Cell. 2016;63(5):898-911. doi:10.1016/j.molcel.2016.06.034 Yan WX, Mirzazadeh R, Garnerone S, et al. BLISS is a versatile and quantitative method for genome-wide profiling of DNA double-strand breaks. Nat Commun. 2017;8(May):1-9. doi:10.1038/ncomms15058
Crosetto N, Mitra A, Silva MJ, et al. Nucleotide-resolution DNA double-strand break mapping by next-generation sequencing. Nat Methods. 2013;10(4):361-365. doi:10.1038/nmeth.2408 Kim D, Lim K, Kim ST, et al. Genome-wide target specificities of CRISPR RNA-guided programmable deaminases. Nat Biotechnol. 2017;35(5):475-480. doi:10.1038/nbt.3852 Lazzarotto CR, Nguyen NT, Tang X, et al. De fi ning CRISPR - Cas9 genome-wide nuclease activities with CIRCLE-seq. 2018;13(November):2615-2642.
Gothe HJ, Bouwman BAM, Gusmao EG, et al. Spatial Chromosome Folding and Active Transcription Drive DNA Fragility and Formation of Oncogenic MLL Translocations. Mol Cell. 2019;75(2):267-283.e12. doi:10.1016/j.molcel.2019.05.015
Bouwman BAM, Agostini F, Garnerone S, et al. Genome-wide detection of DNA double-strand breaks by in-suspension BLISS. Nat Protoc. 2020;15(12):3894-3941. doi:10.1038/s41596-020-0397-2
Jiang F, Doudna JA. CRISPR - Cas9 Structures and Mechanisms. 2017:505-531.
Doudna JA. The promise and challenge of therapeutic genome editing. Nature. 2020;578(7794):229-236. doi:10.1038/s41586-020-1978-5
Tröder SE, Zevnik B (2022) History of genome editing: From meganucleases to CRISPR. Lab Anim. 56(1), 60-68
Li H. (2013) Aligning sequence reads, clone sequences and assembly contigs with BWA-MEM. arXiv:1303.3997v2 [q-bio.GN]
Storey JD, Bass AJ, Dabney A, Robinson D (2022). qvalue: Q-value estimation for false discovery rate control. R package version 2.28.0, http://github.com/jdstorey/qvalue
Donohoue PD, Pacesa M, Lau E, et al. Conformational control of Cas9 by CRISPR hybrid RNA-DNA guides mitigates off-target activity in T cells. Mol Cell. 2021;81(17):3637-3649.e5. doi:10.1016/j.molcel.2021.07.035
Vakulskas CA, Dever DP, Rettig GR, et al. A high-fidelity Cas9 mutant delivered as a ribonucleoprotein complex enables efficient gene editing in human hematopoietic stem and progenitor cells. Nat Med. 2018;24(8):1216-1224. doi:10.1038/s41591-018-0137-0
Chaudhari HG, Penterman J, Whitton HJ, et al. Evaluation of Homology-Independent CRISPR-Cas9 Off-Target Assessment Methods. Cris J. 2020;3(6):440-453. doi:10.1089/crispr.2020.0053
Kim D, Kim S, Kim S, Park J, Kim JS. Genome-wide target specificities of CRISPR-Cas9 nucleases revealed by multiplex Digenome-seq. Genome Res. 2016;26(3):406-415. doi:10.1101/gr.199588.115
Jinek M, Chylinski K, Fonfara I, Hauer M, Doudna JA, Charpentier E. A Programmable Dual-RNA - Guided. 2012;337(August):816-822.
van Overbeek M, Capurso D, Carter MM, et al. DNA Repair Profiling Reveals Nonrandom Outcomes at Cas9-Mediated Breaks. Mol Cell. 2016;63(4):633-646. doi:10.1016/j.mol-cel.2016.06.037
Allen F, Crepaldi L, Alsinet C, et al. Predicting the mutations generated by repair of Cas9-induced double-strand breaks. Nat Biotechnol. 2019;37(1):64-82. doi:10.1038/nbt.4317
Shi X, Shou J, Mehryar MM, et al. Cas9 has no exonuclease activity resulting in staggered cleavage with overhangs and predictable di- and tri-nucleotide CRISPR insertions without template donor. Cell Discov. 2019;5(1):4-7. doi:10.1038/s41421-019-0120-z

## Claims

1. A method for detecting one or more double-strand breaks (DSBs) in DNA comprising steps of
a) providing DNA comprising one or more DSBs,
b) ligating a first linker to the DSB ends to obtain a first linker-DNA conjugate, wherein the first linker comprises a UMI;
c) preferably, removing non-ligated first linkers;
d) enzymatically fragmenting the first linker-DNA conjugates and tagging the obtained DNA fragments with a second linker;
e) PCR-amplifying the tagged DNA fragments to fill gaps in the DNA sequence of these DNA fragments to obtain a first heterotagged population of DNA fragments consisting of DNA fragments flanked by the first linker and the second linker and a second homotagged population of DNA fragments consisting of DNA fragments flanked by two second linkers and not comprising the first linker;
f) enriching the first heterotagged population of DNA fragments by PCR-amplifying and, optionally, size selecting the DNA fragments, wherein, during PCR amplification, a first and a second full-length sequencing adapter is attached to the first and second linker, respectively, at the ends of each DNA fragment of the first heterotagged population of DNA fragments to obtain a sequencing library;
g) sequencing the sequencing library comprising the first heterotagged population of DNA fragments; and
h) bioinformatically analyzing the sequencing results to detect the one or more DNA DSBs.

2. The method of any of claim 1, wherein the one or more DSBs in the DNA have been induced by a genome-editing nuclease, a restriction enzyme, a chemical agent or radiation.

3. The method of any of claim 1 or 2, wherein the one or more DSBs in the DNA have been induced by a genome-editing nuclease selected from the group comprising a CRISPR nuclease, a Meganuclease, a Zinc Finger Nuclease (ZNF), or a transcription activator-like effector nuclease (TALEN).

4. The method of any of the preceding claims, wherein the one or more DSBs in the DNA have been induced by a CRISPR nuclease, wherein the CRISPR nuclease is selected from the group comprising Cas9 and Cas12.

5. The method of any of the preceding claims, wherein the first linker further comprises a first partial sequencing adapter comprising a first sequencing primer binding site and a DNA sequence for attachment of the first full-length sequencing adapter.

6. The method of any of the preceding claims, wherein the second linker comprises a transposase recognition motif and a second partial sequencing adapter comprising a second sequencing primer binding site and a sequence suitable for attachment of the second full-length sequencing adapter.

7. The method of any of the preceding claims, wherein the first linker further comprises a barcode region.

8. The method of any of the preceding claims, wherein the first linker is a double-stranded DNA molecule consisting of a sense DNA strand comprising a nucleic acid sequence having at least 50% sequence identity to SEQ ID NO: 10 and a complementary antisense DNA strand comprising a nucleic acid sequence having at least 50% sequence identity to SEQ ID NO: 11.

9. The method of any of the preceding claims, wherein the second linker comprises a double-stranded transposase recognition motif and a single-stranded overhang and comprises a sense nucleic acid sequence having at least 50% sequence identity to SEQ ID NO: 7.

10. The method of any of the preceding claims, wherein step h) comprises identifying the location of the one or more DSBs in the DNA and/or quantifying the amount of DSBs in the DNA.

11. The method of any of the preceding claims, wherein step h) comprises identifying on- and/or off-targets of a genome-editing nuclease selected from the group comprising a CRISPR nuclease, Meganuclease, Zinc Finger Nuclease (ZNF), or transcription activator-like effector nuclease (TALEN), preferably, of a CRISPR nuclease.

12. The method of claim 11, wherein the genome-editing nuclease is a CRISPR nuclease and step h) comprises
i) filtering for sequencing reads that contain a UMI and, optionally, a barcode;
ii) aligning the filtered sequencing reads to a reference DNA and counting the number of reads representing individual DSBs based on their corresponding UMI and, optionally, barcode;
iii) identifying candidate loci for being CRISPR-edited;
iv) obtaining the sequence context of the candidate loci and keeping only candidate loci comprising a PAM sequence and having a protospacer region exhibiting a desired minimum sequence identity to the sgRNA sequence used to guide the CRISPR nuclease;
v) counting the number of reads representing DSBs within the protospacer region and identifying the cleavage site; and
vi) testing if the enrichment of reads seen in the sequencing library obtained from DNA targeted with a CRISPR nuclease is significant compared to a control library obtained from DNA that has not been targeted with a CRISPR nuclease.

13. A method for determining whether a CRISPR nuclease coupled with a specific gRNA generates a DNA DSB having blunted or a staggered ends, wherein the method comprises steps a) to h) of the method of claim 1, wherein, prior to step b), the method comprises enzymatically blunting the DSB generated by the CRISPR nuclease, and wherein step h) comprises
i) filtering for sequencing reads that contain a UMI and, optionally, a barcode;
ii) aligning the filtered sequencing reads to a reference DNA and counting the number of reads representing individual DSBs based on their corresponding UMI and, optionally, barcode, thereby differentiating between the reads that align on either strand of the reference DNA;
iii) identifying candidate loci for being CRISPR-edited;
iv) obtaining the sequence context of the candidate loci and keeping only candidate loci comprising a PAM sequence and having a protospacer region exhibiting a desired minimum sequence identity to the sgRNA sequence used to guide the CRISPR nuclease;
v) counting the number of reads representing DSBs within the protospacer region and identifying the cleavage site;
vi) testing if the enrichment of reads seen in the sequencing library obtained from DNA targeted with a CRISPR nuclease is significant compared to a control library obtained from DNA that has not been targeted with a CRISPR nuclease;
vii) identifying the presence of nucleotide gaps and/or overlaps, respectively, between sequencing reads starting from the PAM-proximal side and sequencing reads starting from the PAM distal-side of a given cleavage site to determine whether the CRISPR nuclease created a DSB having blunt or staggered break ends; and
viii) counting the number of PAM-proximal sequencing reads representing a staggered scissile profile, and testing if the enrichment of sequencing reads representing a staggered scissile profile seen in the sequencing library obtained from DNA targeted with a CRISPR nuclease is significant compared to a control library obtained from DNA that has not been targeted with a CRISPR nuclease.

14. Use of the method of any of claims 1 to 12 or claim 13 for
(a) profiling the off-target activity of a genome-editing nuclease, preferably, a CRISPR nuclease;
(b) investigating repair kinetics of DSB breaks;
(c) assessing DNA DSB repair capacity by quantifying DSBs in a sample;
(d) detecting off-target activity of base editors;
(e) assessing specificity and fidelity of a CRISPR nuclease; and/or
(f) analysing the ratio of staggered to blunt-ended DSBs induced by a genome-editing nuclease, preferably, a CRISPR nuclease.

15. A kit suitable for performing the method of any of the preceding claims, wherein the kit comprises
a) enzymes suitable for blunting and A-tailing of DNA DSB ends;
b) an enzyme suitable for ligating the first linker to the DNA fragment;
c) a transposase capable of catalyzing tagmentation with the second linker;
d) a set of first linkers;
e) a set of second linkers;
f) nucleotides; and
g) buffers.

16. A computer program product comprising instructions which, when the program is executed by a computer, cause the computer to carry out step h) i) to vi) of the method of claim 12, or step h) i) to viii) of the method of claim 13 and, optionally, any of the uses of claim 14.
